# EUROPEAN PATENT APPLICATION

(11) **EP 4 183 256 A1**
(43) Date of publication of application: **24.05.2023**
(21) Application number: 21842745.8
(22) Date of filing: 13.07.2021
(51) Int. Cl.: A22C 21/00, A61L 101/06, A61L 2/18, A23L 13/00, A23L 13/50, A23B 4/24, A23B 4/26, A01P 3/00, A01N 25/00, A01N 25/02, A01N 59/08

(54) **POULTRY MEAT PRODUCTION METHOD USING CHLOROUS ACID WATER**

(30) Priority: 14.07.2020 JP 2020120838
(71) Applicant: Sankei Co., Ltd., Chuo-ku Osaka-shi Osaka 540-0001 (JP)
(72) Inventor: GODA, Hisataka, Osaka-shi, Osaka 541-0048 (JP)
(74) Representative: Müller-Boré & Partner Patentanwälte PartG mbB
(86) International application number: PCT/JP2021/026315
(87) International publication number: WO 2022/014595

(57) **Abstract**

The present disclosure provides a method for producing chicken meat that is to be consumed raw. The present disclosure particularly provides a method for producing a chicken meat material that can also be used for a chicken meat product that is to be consumed raw, the method comprising a step for bringing chlorous acid water into contact with a chicken carcass. The chlorous acid water contacting step mentioned above includes a step for bringing particulates of chlorous acid water into contact with a chicken carcass. Also provided is a method for producing chicken meat that is to be consumed raw, the method comprising: a step for providing raw chicken meat; and a step for bringing said chicken meat into contact with chlorous acid water to produce chicken meat to be consumed raw. Further provided is chlorous acid water having an average particle size of 1-10 µm.

## Description

### [Technical Field]

The present disclosure relates to a method of manufacturing poultry meat by using a chlorous acid aqueous solution. The present disclosure also relates to a method and apparatus for manufacturing a microparticulate chlorous acid aqueous solution. The present disclosure also relates to a system for sterilizing poultry meat.

### [Background Art]

Campylobacter food poisoning, which occupies the top spot in both the annual number of incidences and the number of patients among bacterial food poisoning in Japan, has become a significant social issue that has shaken the foundation of food safety and resulted in significant economic losses to patients and producers. Chicken meat is known to be contaminated with Campylobacter at a high frequency.

In recent years, there is a preference and trend that deem raw food products to be fresh and tasty. While there is also a demand for raw consumption of meat, even fresh chicken meat contains pathogenic microorganisms including Campylobacter bacteria. Thus, chicken meat for raw consumption that does not result in food poisoning has not been provided.

"Nintei Shokibo Shokucho Shorijo no tameno HACCP no Kangaekata wo Toriireta Eisei Kanri no Tebikisho [Guidebook for sanitary management incorporating the concept of HACCP for certified small-scale poultry processing factories]" mentions enterobacteriaceae as indicator bacteria for strengthening the management of Campylobacter bacteria.

### [Summary of Invention]

### [Solution to Problem]

As a result of diligent studies, the inventors established a method of manufacturing poultry meat such as chicken meat for raw consumption. The present disclosure has been completed by developing a method and apparatus for manufacturing a microparticulate chlorous acid aqueous solution and developing a system for sterilizing poultry meat.

The efficacy of chemical sterilization treatment using a food additive sterilizer: chlorous acid aqueous solution as a measure to prevent food poisoning due to Campylobacter bacteria, which are pathogenic microorganisms that can induce infections, and enterobacteriaceae derived from the intestinal tract, which are indicator bacteria thereof, was investigated under the environment in practice. The specification and standards can be established for poultry meat for raw consumption in order to protect the raw consumption culture unique to Japan.

The present disclosure establishes a "method of manufacturing a chicken meat raw material that can be used for raw consumption". As the background thereof, despite chicken dishes and chicken meat processed products that are raw or nearly raw being provided throughout Japan, there is a lack of not only a raw material that is suitable therefor being provided, but also an established suitable specification or microorganism control standards which can be applicable to raw materials of chicken dishes or chicken meat processed products that are raw or nearly raw. Despite the above, demand for raw consumption of chicken continues to increase, resulting in many food poisoning incidences due to Campylobacter bacteria. As the background reason thereof, chicken meat in distribution which is intended for consumption after heating is provided to consumers in a raw or insufficiently heated condition. However, food poisoning due to Campylobacter bacteria has hardly occurred in Miyazaki prefecture or Kagoshima prefecture. This is because the raw materials used in the local cuisine "chicken tataki" of Miyazaki prefecture and Kagoshima prefecture are manufactured based on the regulations enacted uniquely by the prefectures, and are shipped from only factories that belong to an organization such as the raw consumption conference. However, other prefectures outside of Kyushu have not even discussed such regulations or conditions. Since meat is provided directly, consumers who have consumed raw or nearly raw chicken dishes or chicken meat processed product using such poultry processed meat as a raw material have developed food poisoning caused by Campylobacter bacteria, resulting in a significant issue. Thus, without any measures, the traditional raw chicken consumption culture, which is inherently safe and is a local cuisine of Miyazaki prefecture and Kagoshima prefecture, could be prohibited. The economic loss therefrom would be immeasurable.

Thus, to prevent such a situation, it is necessary to manufacture chicken meat with the number of Campylobacter bacteria reduced to a level where food poisoning due to such bacteria would not occur, and establish microorganism control standards and a system that regulates the standards in order to prevent food poisoning due to Campylobacter bacteria. Chicken meat raw materials produced by such a method should be distributed to the market with a label "for raw consumption". The present disclosure establishes the technologies therefor.

Furthermore, it is known that there are two types of Campylobacter bacteria, i.e., Campylobacter bacteria that can cause food poisoning and Campylobacter bacteria that can cause an infection. In particular, poultry is contaminated with feces during transport from a farm to a processing factory, i.e., poultry arrives at a poultry processing factory while being contaminated with Campylobacter bacteria derived from feces. Since poultry is processed directly in such a state, the Campylobacter bacteria derived from feces propagate and contaminate many facilities and lines within the poultry processing factory. In addition, live fowl is shipped to a poultry processing factory every day. Thus, Campylobacter bacteria of diverse genotypes arriving from various farms settle down and reside in a processing factory resulting in the formation of a unique ecosystem. It is understood that this spreads via humans or objects, resulting in a heightened risk of infection for workers. It is necessary to sterilize such Campylobacter bacteria derived from feces spreading in the poultry processing factory at the early stages of the disassembly process in order to fundamentally improve food poisoning by Campylobacter bacteria, which is currently a problem. For this reason, a pre-processing sterilization step after defeathering is required. Further, thorough reduction of Campylobacter bacteria derived from feces at this stage would be an infection-preventing measure, only after which food poisoning-preventing measure would be established.

The direct cause of food poisoning incidences from Campylobacter bacteria, which is currently a problem, is Campylobacter bacteria derived from the organs. When organs are removed through an evisceration step, intestinal tracks are damaged and the content therein adheres to and contaminates the eviscerated carcass with Campylobacter bacteria derived from the organs. After the evisceration step, the process proceeds to an inside/outside cleaning step and a pre-cooling/main cooling chiller step. During the inside/outside cleaning step, the eviscerated carcass is just washed with a large amount of water. With such a method, stains are cleaned, but Campylobacter bacteria derived from the organs would not be rinsed away. Sodium hypochlorite that is automatically titrated during the pre-cooling/main cooling chiller step is for preventing contamination of chiller water within a chiller tank, and cannot sterilize Campylobacter bacteria derived from the organs adhering to an eviscerated carcass (this is indicated by the data in Table 11 herein). Thus, in order to take food poisoning prevention measures, it is necessary to ensure that Campylobacter bacteria derived from the organs are reduced before the pre-cooling/main cooling chiller steps. Thus, sterilization apparatuses (apparatus B and apparatus C) would be required. Only after deploying such apparatuses, an eviscerated carcass with a Campylobacter bacteria count of 100 or less in terms of the colony count in a quantifying medium, or an MPN value found from an estimated bacteria count which is determined in advance by a method using three sets and three tubes at or below the detection limit (three or less), can be manufactured. Providing and managing a microorganism specification so that the bacteria count at this time can be constantly maintained can enable production of poultry processed meat = poultry meat for raw consumption that can be a raw material of chicken dishes or chicken meat processed products that are raw or nearly raw, which do not result in food poisoning due to Campylobacter bacteria.

Currently, no matter how much an eviscerated carcass is cleaned after an evisceration step, Campylobacter bacteria derived from feces and Campylobacter bacteria derived from organs coexist. For this reason, an effective measure could not be taken for the problem of food poisoning due to Campylobacter bacteria. Moreover, the lack of specialized knowledge leads to a misunderstanding, where an eviscerated carcass would be cleaned by providing an inside/outside cleaning step, and sodium hypochlorite in a pre-cooling/main cooling chiller has a sterilization effect. It is obvious that food poisoning due to Campylobacter bacteria would occur if a chicken dish or chicken meat processed product that is prepared raw or nearly raw by using an eviscerated carcass manufactured under such an environment without heating is consumed. In view of such a background, it is understood that food poisoning measures must be taken by first installing both a pre-treatment sterilization apparatus (apparatus A) and main sterilization apparatuses (apparatus B and apparatus C) to suppress Campylobacter bacteria derived from feces as an infection preventing measure in order to prevent food poisoning.

For example, the present invention provides the following items.

### (Item 1)

A method of manufacturing poultry meat, comprising a step of contacting a chlorous acid aqueous solution with a meat raw material of a fowl for consumption.

### (Item 1A)

The method of item 1, wherein the fowl comprise a carcass thereof or a part thereof.

### (Item 2)

The method of the preceding items, comprising a step of contacting a chlorous acid aqueous solution with the fowl after removing an organ from the fowl.

### (Item 3)

The method of any one of the preceding items, further comprising a step of contacting a chlorous acid aqueous solution after defeathering the fowl and before removing an organ from the fowl.

### (Item 4)

The method of any one of the preceding items, further comprising a step of contacting a chlorous acid aqueous solution after removing an organ from the fowl, contacting a chlorous acid aqueous solution with the fowl, and cleaning inside and outside of the fowl with a chlorous acid aqueous solution supplemented solution and before cooling the fowl.

### (Item 5)

The method of any one of the preceding items, further comprising a step of contacting a chlorous acid aqueous solution after cooling the fowl and before refrigeration.

### (Item 6)

The method of any one of the preceding items, comprising a step of cleaning inside and outside of the fowl, wherein the step of cleaning inside and outside of the fowl comprises a step of sterilizing a microorganism derived from an organ while rinsing off organ content adhering to a surface with a chlorous acid aqueous solution having a free chlorine concentration of 1 to 200 mg/L.

### (Item 7)

The method of any one of the preceding items, wherein a microorganism adhering to a surface of a fowl is sterilized with a chlorous acid aqueous solution while cooling.

### (Item 8)

The method of any one of the preceding items, wherein a free chlorine concentration of a chlorous acid aqueous solution in the cooling is 1 to 200 mg/L.

### (Item 9)

The method of any one of the preceding items, wherein the step of contacting a chlorous acid aqueous solution also comprises a step of contacting a microparticulate chlorous acid aqueous solution.

### (Item 10)

The method of any one of the preceding items, wherein the step of contacting a chlorous acid aqueous solution comprises a step of passing the fowl through a space where a microparticulate chlorous acid aqueous solution is accumulated.

### (Item 11)

The method of any one of the preceding items, wherein a mean particle size of the microparticulate chlorous acid aqueous solution is 1 um to 10 µm.

### (Item 12)

The method of any one of the preceding items, wherein a free chlorine concentration of the chlorous acid aqueous solution is 1 mg/L to 200 mg/L.

### (Item 13)

The method of any one of the preceding items, wherein the poultry meat is poultry meat that can be used not only as a conventional chicken meat raw material, but also as a raw material of a chicken meat product for raw consumption.

### (Item 14)

The method of any one of the preceding items, wherein the fowl are a parent stock (spent hen), broiler, roaster, or free range chicken.

### (Item 15)

The method of any one of the preceding items, wherein the method results in enterobacteriaceae adhering to the poultry meat which is negative in 25 g of a final product, Campylobacter bacteria which are negative according to a quantification method (mCCDA (SEL) medium method) and 50 MPN or less in 100 g of a final product according to a method using three sets and three tubes, Salmonella bacteria which are 0.014 cfu/g or less, and enterohemorrhagic E. coli which is 0.014 cfu/g or less.

### (Item 16)

The method of any one of the preceding items, wherein the enterobacteriaceae is a group of bacteria revealed based on a testing method according to the ISO methodology (21528).

### (Item 17)

The method of any one of the preceding items, wherein the Campylobacter bacteria are C. coli, C. concisus, C. curvus, C. fetus, C. gracilis, C. helveticus, C. hominis, C. hyointestinalis, C. insulaenigrae, C. jejuni, C. lanienae, C. lari, C. mucosalis, C. rectus, C. showae, C. sputorum, or C. upsaliensis.

### (Item 18)

The method of any one of the preceding items, wherein the Salmonella bacteria are S. abortusequi, S. abortusovis, S. typhisuis, S. pullorum, S. gallinarum, S. abortusbovis, S. typhi, S. paratyphi A, S. paratyphi B, S. Bongori, S. typhimurium, S. enteritidis, S. choleraesuis, S. sendai, S. oranienburg, S. chester, or S. arizonae.

### (Item 19)

The method of any one of the preceding items, wherein the enterohemorrhagic E. coli is E. coli O157, E. coli O26, or E. coli O111.

### (Item 20)

The method of any one of the preceding items, wherein a step of contacting a chlorous acid aqueous solution is performed in every step of the steps.

### (Item 21)

A method of producing poultry meat, comprising:
a step of providing meat of a fowl for consumption; and
a step of contacting the meat of a fowl with a chlorous acid aqueous solution to produce poultry meat.

### (Item 22)

The method of any one of the preceding items, wherein the method results in enterobacteriaceae adhering to the poultry meat which is negative in 25 g of a final product, Campylobacter bacteria which are negative according to a quantification method (mCCDA (SEL) medium method) and 50 MPN or less in 100 g of a final product according to a method using three sets and three tubes, Salmonella bacteria which are 0.014 cfu/g or less, and enterohemorrhagic E. coli which is 0.014 cfu/g or less.

### (Item 23)

The method of any one of the preceding items, wherein the enterobacteriaceae is a group of bacteria revealed based on a testing method according to the ISO methodology (21528).

### (Item 24)

The method of any one of the preceding items, wherein the Campylobacter bacteria are C. coli, C. concisus, C. curvus, C. fetus, C. gracilis, C. helveticus, C. hominis, C. hyointestinalis, C. insulaenigrae, C. jejuni, C. lanienae, C. lari, C. mucosalis, C. rectus, C. showae, C. sputorum, or C. upsaliensis.

### (Item 25)

The method of any one of the preceding items, wherein the Salmonella bacteria are S. abortusequi, S. abortusovis, S. typhisuis, S. pullorum, S. gallinarum, S. abortusbovis, S. typhi, S. paratyphi A, S. paratyphi B, S. Bongori, S. typhimurium, S. enteritidis, S. choleraesuis, S. sendai, S. oranienburg, S. chester, or S. arizonae.

### (Item 26)

The method of any one of the preceding items, wherein the enterohemorrhagic E. coli is E. coli O157, E. coli O26, or E. coli O111.

### (Item 27)

The method of any one of the preceding items, wherein the poultry meat is poultry meat that can be used not only as a conventional chicken meat raw material, but also as a raw material of a chicken meat product for raw consumption.

### (Item 28)

A method of providing a poultry meat dish comprising a step of preparing a poultry meat dish using poultry meat produced by the method of any one of the preceding items.

### (Item 29)

A chlorous acid aqueous solution having a mean particle size of 1 um to 10 µm.

### (Item 30)

A sterilizing agent comprising a microparticulate chlorous acid aqueous solution.

### (Item 31)

The sterilizing agent of any one of the preceding items, wherein the sterilizing agent is for sterilizing Campylobacter bacteria and enterobacteriaceae.

### (Item 32)

The sterilizing agent of any one of the preceding items, wherein the Campylobacter bacteria are C. coli, C. concisus, C. curvus, C. fetus, C. gracilis, C. helveticus, C. hominis, C. hyointestinalis, C. insulaenigrae, C. jejuni, C. lanienae, C. lari, C. mucosalis, C. rectus, C. showae, C. sputorum, or C. upsaliensis.

### (Item 33)

The method of any one of the preceding items, wherein the enterobacteriaceae is a group of bacteria revealed based on a testing method according to the ISO methodology (21528).

### (Item 34)

A method of manufacturing a microparticulate chlorous acid aqueous solution by supplying a chlorous acid aqueous solution at an amount of 0.1 L/hour to 10.0 L/hour to a nozzle having a diameter of 5 um to 15 µm, at an amount of air of 10 L/minute to 60 L/minute and at a pressure of 0.04 MPa to 1.2 MPa.

### (Item 35)

The method of any one of the preceding items, wherein an available concentration of a chlorous acid aqueous solution is 1 mg/L to 50 mg/L.

### (Item 36)

The method of any one of the preceding items, wherein a spraying time is 2 seconds to 30 seconds.

### (Item 37)

The method of any one of the preceding items, wherein a mean particle size of a chlorous acid aqueous solution is 8 um or less.

### (Item 38)

The method of any one of the preceding items, wherein a shape of a microparticle of the chlorous acid aqueous solution is round, oval, diamond, or omnidirectional.

### (Item 39)

The method of any one of the preceding items, wherein a spraying speed is 0.39 L/minute to 3.2 L/minute.

### (Item 40)

The method of any one of the preceding items, wherein a spraying speed is 0.9 L/hour to 2.0 L/hour.

### (Item 41)

An apparatus for manufacturing a microparticulate chlorous acid aqueous solution, comprising a nozzle having a diameter of 1 um to 10 um and means for providing a chlorous acid aqueous solution at an amount of 0.1 L/hour to 10.0 L/hour, at an amount of air of 10 L/minute to 60 L/minute and at a pressure of 0.04 MPa to 1.2 MPa.

### (Item 42)

The apparatus of any one of the preceding items, wherein an available concentration of a chlorous acid aqueous solution is 1 mg/L to 50 mg/L.

### (Item 43)

The apparatus of any one of the preceding items, wherein a spraying time is 2 seconds to 30 seconds.

### (Item 44)

The apparatus of any one of the preceding items, wherein a mean particle size of a chlorous acid aqueous solution is 8 um or less.

### (Item 45)

The apparatus of any one of the preceding items, wherein a shape of a microparticle of the chlorous acid aqueous solution is round, oval, diamond, or omnidirectional.

### (Item 46)

The apparatus of any one of the preceding items, wherein a spraying speed is 0.39 L/minute to 3.2 L/minute.

### (Item 47)

The apparatus of any one of the preceding items, wherein a spraying speed is 0.9 L/hour to 2.0 L/hour.

### (Item 48)

A system for sterilizing meat of a fowl for consumption, comprising:
a spray nozzle for spraying a chlorous acid aqueous solution;
an instrument for providing the meat of a fowl;
means for moving the instrument; and
a barrier for forming a processing space for blocking a side surface with respect to a direction of movement of the instrument.

### (Item 49)

The system of any one of the preceding items, further comprising a spraying zone for spraying a chlorous acid aqueous solution.

### (Item 50)

The system of any one of the preceding items, wherein the spray nozzle provides a chlorous acid aqueous solution having a mean particle size of 1 um to 10 µm.

### (Item 51)

The system of any one of the preceding items, wherein the sterilization of the meat of a fowl is sterilization of poultry meat that can be used not only as a conventional chicken meat raw material, but also as a raw material of a chicken meat product for raw consumption.

### (Item 52)

A system for manufacturing poultry meat, comprising:
at least one unit selected from the group consisting of a blood-letting unit, a scalding unit, a defeathering unit, an evisceration unit, an inside/outside cleaning unit, a cooling unit, and a refrigeration unit; and
a unit or means for contacting a chlorous acid aqueous solution.

### (Item 53)

The system of any one of the preceding items, wherein the unit for contacting a chlorous acid aqueous solution comprises a unit for spraying a chlorous acid aqueous solution.

### (Item 54)

The system of any one of the preceding items, wherein the unit for contacting a chlorous acid aqueous solution comprises a unit comprising a chlorous acid aqueous solution supplemented solution.

### (Item 55)

The system of any one of the preceding items, wherein cleaning water used in the inside/outside cleaning unit comprises a chlorous acid aqueous solution.

### (Item 56)

The system of any one of the preceding items, wherein cooling water used in the cooling unit comprises a chlorous acid aqueous solution.

### (Item 57)

The system of any one of the preceding items, wherein the poultry meat can also be used as a raw material of a chicken meat product for raw consumption.

### (Item 58)

A method of sterilizing poultry meat, comprising a step of contacting a chlorous acid aqueous solution with a meat raw material of a fowl for consumption.

### (Item 59)

The method of any one of the preceding items, comprising a step of contacting a chlorous acid aqueous solution with the fowl after removing an organ from the fowl.

### (Item 60)

The method of any one of the preceding items, further comprising a step of contacting a chlorous acid aqueous solution after defeathering the fowl and before removing an organ from the fowl.

### (Item 61)

The method of any one of the preceding items, further comprising a step of contacting a chlorous acid aqueous solution after removing an organ from the fowl, contacting a chlorous acid aqueous solution with the fowl, and cleaning inside and outside of the fowl with a chlorous acid aqueous solution supplemented solution and before cooling the fowl.

### (Item 62)

The method of any one of the preceding items, further comprising a step of contacting a chlorous acid aqueous solution after cooling the fowl and before refrigeration.

### (Item 63)

The method of any one of the preceding items, comprising a step of cleaning inside and outside of the fowl, wherein the step of cleaning inside and outside of the fowl comprises a step of sterilizing a microorganism derived from an organ while rinsing off organ content adhering to a surface with a chlorous acid aqueous solution having a free chlorine concentration of 1 to 200 mg/L.

### (Item 64)

The method of any one of the preceding items, wherein a microorganism adhering to a surface of a fowl is sterilized with a chlorous acid aqueous solution while cooling.

### (Item 65)

The method of any one of the preceding items, wherein a free chlorine concentration of a chlorous acid aqueous solution in the cooling is 1 to 200 mg/L.

### (Item 66)

The method of any one of the preceding items, wherein the step of contacting a chlorous acid aqueous solution comprises a step of contacting a microparticulate chlorous acid aqueous solution.

### (Item 67)

The method of any one of the preceding items, wherein the step of contacting a chlorous acid aqueous solution comprises a step of passing the fowl through a space where a microparticulate chlorous acid aqueous solution is accumulated.

### (Item 68)

The method of any one of the preceding items, wherein a mean particle size of the microparticulate chlorous acid aqueous solution is 1 um to 10 µm.

### (Item 69)

The method of any one of the preceding items, wherein a free chlorine concentration of the chlorous acid aqueous solution is 1 mg/L to 200 mg/L.

### (Item 70)

The method of any one of the preceding items, wherein the poultry meat is poultry meat that can be used not only as a conventional chicken meat raw material, but also as a raw material of a chicken meat product for raw consumption.

### (Item 71)

The method of any one of the preceding items, wherein the fowl are a parent stock (spent hen), broiler, roaster, or free range chicken.

### (Item 72)

The method of any one of the preceding items, wherein the method results in enterobacteriaceae adhering to the poultry meat which is negative in 25 g of a final product, Campylobacter bacteria which are negative according to a quantification method (mCCDA (SEL) medium method) and 50 MPN or less in 100 g of a final product according to a method using three sets and three tubes, Salmonella bacteria which are 0.014 cfu/g or less, and enterohemorrhagic E. coli which is 0.014 cfu/g or less.

### (Item 73)

The method of any one of the preceding items, wherein the enterobacteriaceae is a group of bacteria revealed based on a testing method according to the ISO methodology (21528).

### (Item 74)

The method of any one of the preceding items, wherein the Campylobacter bacteria are C. coli, C. concisus, C. curvus, C. fetus, C. gracilis, C. helveticus, C. hominis, C. hyointestinalis, C. insulaenigrae, C. jejuni, C. lanienae, C. lari, C. mucosalis, C. rectus, C. showae, C. sputorum, or C. upsaliensis.

### (Item 75)

The method of any one of the preceding items, wherein the Salmonella bacteria are S. abortusequi, S. abortusovis, S. typhisuis, S. pullorum, S. gallinarum, S. abortusbovis, S. typhi, S. paratyphi A, S. paratyphi B, S. Bongori, S. typhimurium, S. enteritidis, S. choleraesuis, S. sendai, S. oranienburg, S. chester, or S. arizonae.

### (Item 76)

The method of any one of the preceding items, wherein the enterohemorrhagic E. coli is E. coli O157, E. coli O26, or E. coli O111.

### (Item 77)

The method of any one of the preceding items, wherein the Campylobacter bacteria and enterobacteriaceae comprise those derived from feces.

### (Item 78)

The method of any one of the preceding items, wherein the Campylobacter bacteria and enterobacteriaceae comprise those derived from an organ.

### (Item 79)

The method of any one of the preceding items, wherein a step of contacting a chlorous acid aqueous solution is performed in every step of the steps.

### (Item 80)

An apparatus for contacting a chlorous acid aqueous solution after defeathering a fowl and before removing an organ from the fowl.

### (Item 81)

An apparatus for contacting a chlorous acid aqueous solution with a fowl after removing an organ from the fowl.

### (Item 82)

An apparatus for contacting a chlorous acid aqueous solution after removing an organ from a fowl, contacting a chlorous acid aqueous solution with the fowl, and cleaning inside and outside of the fowl with a chlorous acid aqueous solution supplemented solution and before cooling the fowl.

### (Item 83)

An apparatus for contacting a chlorous acid aqueous solution after cooling the fowl and before refrigeration.

### (Item 84)

The apparatus of any one of the preceding items, wherein the chlorous acid aqueous solution is a microparticulate chlorous acid aqueous solution.

### (Item 85)

The apparatus of any one of the preceding items, wherein the fowl pass through a space where a microparticulate chlorous acid aqueous solution is accumulated.

### (Item 86)

The apparatus of any one of the preceding items, wherein a mean particle size of the microparticulate chlorous acid aqueous solution is 1 um to 10 µm.

### (Item 87)

The apparatus of any one of the preceding items, wherein a free chlorine concentration of the chlorous acid aqueous solution is 1 mg/L to 200 mg/L.

### (Item 88)

A system comprising one or more of the apparatuses of any one of the preceding items.

### (Item 89)

A system for sterilizing poultry meat, comprising an apparatus for contacting a chlorous acid aqueous solution with a meat raw material of a fowl for consumption.

### (Item 90)

The system of any one of the preceding items, comprising an apparatus for contacting a chlorous acid aqueous solution with the fowl after removing an organ from the fowl.

### (Item 91)

The system of any one of the preceding items, further comprising an apparatus for contacting a chlorous acid aqueous solution after defeathering the fowl and before removing an organ from the fowl.

### (Item 92)

The system of any one of the preceding items, further comprising an apparatus for contacting a chlorous acid aqueous solution after removing an organ from the fowl, contacting a chlorous acid aqueous solution with the fowl, and cleaning inside and outside of the fowl with a chlorous acid aqueous solution supplemented solution and before cooling the fowl.

### (Item 93)

The system of any one of the preceding items, further comprising an apparatus for contacting a chlorous acid aqueous solution after cooling the fowl and before refrigeration.

### (Item 94)

The system of any one of the preceding items, wherein cleaning inside and outside of the fowl sterilizes a microorganism derived from an organ while rinsing off organ content adhering to a surface with a chlorous acid aqueous solution having a free chlorine concentration of 1 to 200 mg/L.

### (Item 95)

The system of any one of the preceding items, wherein a microorganism adhering to a surface of a fowl is sterilized with a chlorous acid aqueous solution while cooling.

### (Item 96)

The system of any one of the preceding items, wherein a free chlorine concentration of a chlorous acid aqueous solution in the cooling is 1 to 200 mg/L.

### (Item 97)

The system of any one of the preceding items for contacting a microparticulate chlorous acid aqueous solution.

### (Item 98)

The system of any one of the preceding items, wherein the fowl pass through a space where a microparticulate chlorous acid aqueous solution is accumulated.

### (Item 99)

The system of any one of the preceding items, wherein a mean particle size of the microparticulate chlorous acid aqueous solution is 1 um to 10 µm.

### (Item 100)

The system of any one of the preceding items, wherein a free chlorine concentration of the chlorous acid aqueous solution is 1 mg/L to 200 mg/L.

### (Item 101)

The system of any one of the preceding items, wherein the poultry meat is poultry meat that can be used not only as a conventional chicken meat raw material, but also as a raw material of a chicken meat product for raw consumption.

### (Item 102)

The system of any one of the preceding items, wherein the fowl are a parent stock (spent hen), broiler, roaster, or free range chicken.

### (Item 103)

The system of any one of the preceding items, wherein the method results in enterobacteriaceae adhering to the poultry meat which is negative in 25 g of a final product, Campylobacter bacteria which are negative according to a quantification method (mCCDA (SEL) medium method) and 50 MPN or less in 100 g of a final product according to a method using three sets and three tubes, Salmonella bacteria which are 0.014 cfu/g or less, and enterohemorrhagic E. coli which is 0.014 cfu/g or less.

### (Item 104)

The system of any one of the preceding items, wherein the enterobacteriaceae is a group of bacteria revealed based on a testing method according to the ISO methodology (21528).

### (Item 105)

The system of any one of the preceding items, wherein the Campylobacter bacteria are C. coli, C. concisus, C. curvus, C. fetus, C. gracilis, C. helveticus, C. hominis, C. hyointestinalis, C. insulaenigrae, C. jejuni, C. lanienae, C. lari, C. mucosalis, C. rectus, C. showae, C. sputorum, or C. upsaliensis.

### (Item 106)

The system of any one of the preceding items, wherein the Salmonella bacteria are S. abortusequi, S. abortusovis, S. typhisuis, S. pullorum, S. gallinarum, S. abortusbovis, S. typhi, S. paratyphi A, S. paratyphi B, S. Bongori, S. typhimurium, S. enteritidis, S. choleraesuis, S. sendai, S. oranienburg, S. chester, or S. arizonae.

### (Item 107)

The system of any one of the preceding items, wherein the enterohemorrhagic E. coli is E. coli O157, E. coli O26, or E. coli O111.

### (Item 108)

The system of any one of the preceding items, wherein the Campylobacter bacteria and enterobacteriaceae comprise those derived from feces.

### (Item 109)

The system of any one of the preceding items, wherein the Campylobacter bacteria and enterobacteriaceae comprise those derived from an organ.

### (Item 110)

Use of a chlorous acid aqueous solution for the manufacture of poultry meat.

### (Item 111)

Use of a chlorous acid aqueous solution for the manufacture of poultry meat that can be consumed raw.

### (Item 112)

Use of a chlorous acid aqueous solution for sterilizing poultry meat.

The present disclosure is intended so that one or more of the features described above can be provided not only as the explicitly disclosed combinations, but also as other combinations. Additional embodiments and advantages of the present disclosure are recognized by those skilled in the art by reading and understanding the following detailed description as needed.

### [Advantageous Effects of Invention]

Pathogenic microorganisms were able to be reduced to a bacteria count where food poisoning does not occur or lower in poultry meat. This enables distribution of chicken meat for raw consumption.

### [Brief Description of Drawings]

[Figure 1] Figure **1** shows a schematic flowchart of the poultry processing steps in Example 1.
[Figure 2] Figure **2** shows a picture of a 5 cm × 5 cm chicken skin observed through an optical microscope, which was immersed in a space filled with microparticles of inkjet ink (cyan) with a size of 8 um or less in a plastic case, left standing for one minute, and then sliced. The picture shows that the inkjet ink has contacted and permeated through the chicken skin.
[Figure 3] Figure **3** shows the change in Campylobacter bacteria due to spraying of microparticles of chlorous acid aqueous solution and comparison of each season throughout a year.
[Figure 4] Figure **4** shows the change in enterobacteriaceae due to spraying of microparticles of a chlorous acid aqueous solution.
[Figure 5] Figure **5** is a schematic diagram of a cooling water recovery method of Example 1.
[Figure 6] Figure **6** shows the change in the available chlorine concentration and free chlorine concentration and turbidity at point A in Example 2.
[Figure 7] Figure **7** shows the change in the available chlorine concentration and free chlorine concentration and turbidity at point B in Example 2.

### [Description of Embodiments]

The present disclosure is described in more detail hereinafter. Throughout the entire specification, a singular expression should be understood as encompassing the concept thereof in the plural form, unless specifically noted otherwise. Thus, singular articles (e.g., "a", "an", "the", and the like in case of English) should also be understood as encompassing the concept thereof in the plural form, unless specifically noted otherwise. The terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

The abbreviations used herein have the conventional meaning within the scope of the art, unless specifically noted otherwise.

The term "about" for a value or parameter herein includes variations about the value or parameter itself. Unless specifically noted otherwise, "about X" for example includes "X" itself as well as values with an acceptable error of ± 10% therefrom.

As used herein, "chlorous acid aqueous solution" is an aqueous solution comprising chlorous acid (HClO₂) used as a sterilizing agent, which can stably maintain chlorous acid (HClO₂) over a long period of time. The presence of a chlorous acid aqueous solution can be confirmed if an absorbent section representing a peak near 260 nm and an absorbent section representing a peak near 350 nm can be simultaneously observed between wavelengths of 240 to 420 nm in the UV spectrum, i.e., if a double peak is exhibited, when a chlorous acid aqueous solution sample is measured with a spectrophotometer.

A chlorous acid aqueous solution can be prepared by the methods disclosed in International Publication No. WO 2008/026607, WO 2014/188310, WO 2014/188311, WO 2014/188312, WO 2015/093062, or WO 2017/170904.

"Chlorous acid aqueous solution" is a sterilizer that was designated as a food additive on February 1, 2013 and has chlorous acid (HClO₂) as the primary active ingredient. The primary active ingredient chlorous acid (HClO₂) of such a "chlorous acid aqueous solution" is a semi-stable chemical substance, which is approved by the USDA and FDA as a food additive: processing aid as an especially safe substance.

Moreover, "chlorous acid aqueous solution" can exert a potent sterilization effect even in the presence of organic matter. Chlorous acid aqueous solution was highly praised as "only chlorous acid aqueous solution was capable of inactivation to the detection limit or below under all load conditions" in "Heisei 27 Nendo Norouirusu no Fukatsuka Joken ni Kansuru Chosa [2015 Investigation on Norovirus Inactivation Conditions]" at the National Institute of Health Sciences (NIHS). With the revisions to the Ordinance for Enforcement of the Food Sanitation Act, listing of chlorous acid aqueous solutions is ongoing in "Tairyo Chori Shisetsu Chori Manyuaru [Manual for Food Preparation at Large-scale Food Preparation Facilities]" and "Tsukemono no Eisei Kihan [Code of Hygienic Practice for Pickles]", etc., in order of incidents of large scale food poisoning.

In addition, an application of chlorous acid aqueous solutions for a pharmaceutical product as a class II sterilizing disinfectant is pending. With this premise, chlorous acid aqueous solution is added to guidelines such as "Hoikusho ni okeru Kansensho Taisaku Gaidorain [Infection Control Measure Guidelines at Nursery Center]" under the administration of the Ministry of Health, Labour and Welfare including "Norouirusu ni Kansuru Q&A [Q&A regarding noroviruses]", relevant manuals such as "Koreisha Kaigo Shien Shisetsu ni okeru Kansen Taisaku Manyuaru [Manual for Controlling Infection at Elderly Care Support Facility]", various sanitation codes such as "Bento/Sozai no Eisei Kihan [Code of Sanitation for Bento/Dishes]", etc., which are also being revised as needed. A chlorous acid aqueous solution is a substance that is supplied to wide-ranging markets in food sanitation and environmental sanitation markets in Japan.

"Chlorous acid aqueous solution" with chlorous acid as the primary active ingredient possesses the same or more potent sterilizing power than "hypochlorous acid water" or "sodium hypochlorite". Meanwhile, a "chlorous acid aqueous solution" having a characteristic of possessing gradual reactivity with no instantaneous sterilization effect (immediate effect), but gradual reactivity while having a precise as well as sustained stable sterilizing power can exert a sterilization effect that is precise and accurate, albeit slowly, under a contaminated environment with a large quantity of organic matters, which had been considered as the most challenging for chlorine oxide based agents (sterilizing power against microorganisms that are latent in contamination).

For this reason, a chlorous acid aqueous solution can exert an inactivation effect on resistant bacteria (heat-resistant bacteria with increased resistance by forming spores, drug-resistant bacteria against which antibiotics are no longer effective, etc.), fungi such as mold or yeast, and viruses (including both enveloped viruses and non-enveloped viruses), which have been difficult to sterilize. "Chlorous acid aqueous solution" does not need to be prepared upon use or require a dedicated generator, etc. A chlorous acid aqueous solution is safe and can be used anywhere by anyone whenever use thereof is desirable.

In addition, the effect of "chlorous acid aqueous solution" in the presence of an organic matter is listed in "Heisei 27 Nendo Norouirusu no Fukatsuka Joken ni Kansuru Chosa Hokokusho [2015 Investigative Report on Norovirus Inactivation Conditions] (National Institute of Health Sciences, Division of Biomedical Food Research)" on the website of the Ministry of Health, Labour and Welfare.

As used herein, "food poisoning" refers to oral infection resulting in a symptom (case) through ingestion of food (oral intake). Food poisoning is a type of infection, which is a food-mediated infection. An infection is induced by a microorganism that is present in an animal (livestock) or nature. Intake of meat, etc. results in many food poisoning incidents every year. Microbial food poisoning includes infectious food poisoning such as invasive type (Salmonella, etc.) and toxigenic type (Vibrio parahaemolyticus, pathogenic E. coli, etc.), and toxigenic food poisoning from Staphylococcus aureus, botulism, etc. Examples of viral food poisoning include food poisoning due to a norovirus, hepatitis A virus, hepatitis E virus, etc. Examples of chemical food poisoning include food poisoning due to a hazardous food additive (arsenic, residual pesticide, etc.), contaminant, etc. Examples of natural food poisoning include animal-derived food poisoning due to a natural toxin such as puffer fish toxin and shellfish toxin, and vegetable- derived food poisoning due to natural toxins such as poison mushrooms and poisonous plants. Examples of food poisoning also include allergic food poisoning.

As used herein, "pathogenic microorganism" refers to microorganisms infecting through a human or environment, excluding food poisoning (oral infection). Infected patients can disperse microorganisms through diarrhea, vomit, cough, etc. to cause infections through secondary infection.

As used herein, "free chlorine", "free chlorine concentration", or "free residual chlorine concentration" is a value that is measured according to Appendix 3 in "Testing method for free residual chlorine and bound chlorine specified by the Minister of Health, Labour and Welfare based on the stipulation of Article 17(2) of the regulation of the Water Supply Act" (hereinafter, colorimetry (DPD indicator)), and is a value obtained by oxidation of a DPD indicator.

As used herein, "raw consumption" refers to consumption in a non-cooked state (raw).

As used herein, "blood-letting" refers to removal of blood of a chicken from the body.

As used herein, "defeathering" refers to removal of feathers from a chicken.

As used herein, "evisceration" refers to removal of the organs from a chicken carcass.

As used herein, "inside/outside cleaning" refers to cleaning the inside and outside of a chicken carcass whose organs have been removed.

As used herein, "microparticle" refers to a particle with a mean particle size that is small to the extent that a hand would not be wet upon contact. Such a mean particle size can be 1 um to 10 µm.

As used herein, "a fowl for consumption" refers to a fowl that is suitable for consumption, referring to the entire body of such a fowl or a part thereof. Examples thereof include the carcass of a fowl and parts thereof. This refers to raw materials that can be consumed such as meat, organs, and bones.

As used herein, "group of bacteria revealed based on a testing method according to the ISO methodology (21528)" refers to enterobacteriaceae microorganisms which are revealed when tested in accordance with the ISO methodology (21528). Such microorganisms can be oxidase reaction negative microorganisms, which form a characteristic colony on a violet red bile glucose (VRBG) agar medium and ferment glucose upon testing. Examples thereof include coliform bacteria, Salmonella, Shigella, Yersinia, etc.

### (Preferred Embodiments)

One aspect of the present disclosure provides a method of manufacturing poultry meat, comprising a step of contacting a chlorous acid aqueous solution with a meat raw material of a fowl for consumption. The method can be used in the manufacture of poultry meat for raw consumption at a food factory, etc.

The present disclosure also provides a method comprising a step of contacting a chlorous acid aqueous solution with the fowl after removing an organ from the fowl.

The present disclosure also provides a method further comprising a step of contacting a chlorous acid aqueous solution after defeathering the fowl and before removing an organ from the fowl.

The present disclosure also provides a method further comprising a step of contacting a chlorous acid aqueous solution after removing an organ from the fowl, contacting a chlorous acid aqueous solution with the fowl, and cleaning inside and outside of the fowl with a chlorous acid aqueous solution supplemented solution and before cooling the fowl.

The present disclosure also provides a method further comprising a step of contacting a chlorous acid aqueous solution after cooling the fowl and before refrigeration.

The present disclosure also provides a method, wherein the step of cleaning inside and outside of the fowl comprises a step of sterilizing a microorganism derived from an organ while rinsing off organ content adhering to a surface with a chlorous acid aqueous solution having a free chlorine concentration of 1 to 200 mg/L. The free chlorine concentration therein can be 1 mg/L or greater, 5 mg/L or greater, 10 mg/L or greater, 15 mg/L or greater, 20 mg/L or greater, 25 mg/L or greater, 30 mg/L or greater, 35 mg/L or greater, 40 mg/L or greater, 45 mg/L or greater, 50 mg/L or greater, 55 mg/L or greater, 60 mg/L or greater, 65 mg/L or greater, 70 mg/L or greater, 75 mg/L or greater, 80 mg/L or greater, 85 mg/L or greater, 90 mg/L or greater, 95 mg/L or greater, 100 mg/L or greater, 110 mg/L or greater, 120 mg/L or greater, 130 mg/L or greater, 140 mg/L or greater, 150 mg/L or greater, 160 mg/L or greater, 170 mg/L or greater, 180 mg/L or greater, or 190 mg/L or greater. The free chlorine concentration therein can be 200 mg/L or less, 190 mg/L or less, 180 mg/L or less, 170 mg/L or less, 160 mg/L or less, 150 mg/L or less, 140 mg/L or less, 130 mg/L or less, 120 mg/L or less, 110 mg/L or less, 100 mg/L or less, 95 mg/L or less, 90 mg/L or less, 85 mg/L or less, 80 mg/L or less, 75 mg/L or less, 70 mg/L or less, 65 mg/L or less, 60 mg/L or less, 55 mg/L or less, 50 mg/L or less, 45 mg/L or less, 40 mg/L or less, 35 mg/L or less, 30 mg/L or less, 25 mg/L or less, 20 mg/L or less, 15 mg/L or less, 10 mg/L or less, or 5 mg/L or less. The free chlorine concentration therein can be within the range of any combination between these values.

The present disclosure also provides a method of sterilizing a microorganism adhering to a surface of a fowl with a chlorous acid aqueous solution while cooling.

The present disclosure also provides a method, wherein a free chlorine concentration of a chlorous acid aqueous solution in the cooling water is 1 to 200 mg/L. The free chlorine concentration therein can be 1 mg/L or greater, 5 mg/L or greater, 10 mg/L or greater, 15 mg/L or greater, 20 mg/L or greater, 25 mg/L or greater, 30 mg/L or greater, 35 mg/L or greater, 40 mg/L or greater, 45 mg/L or greater, 50 mg/L or greater, 55 mg/L or greater, 60 mg/L or greater, 65 mg/L or greater, 70 mg/L or greater, 75 mg/L or greater, 80 mg/L or greater, 85 mg/L or greater, 90 mg/L or greater, 95 mg/L or greater, 100 mg/L or greater, 110 mg/L or greater, 120 mg/L or greater, 130 mg/L or greater, 140 mg/L or greater, 150 mg/L or greater, 160 mg/L or greater, 170 mg/L or greater, 180 mg/L or greater, or 190 mg/L or greater. The free chlorine concentration therein can be 200 mg/L or less, 190 mg/L or less, 180 mg/L or less, 170 mg/L or less, 160 mg/L or less, 150 mg/L or less, 140 mg/L or less, 130 mg/L or less, 120 mg/L or less, 110 mg/L or less, 100 mg/L or less, 95 mg/L or less, 90 mg/L or less, 85 mg/L or less, 80 mg/L or less, 75 mg/L or less, 70 mg/L or less, 65 mg/L or less, 60 mg/L or less, 55 mg/L or less, 50 mg/L or less, 45 mg/L or less, 40 mg/L or less, 35 mg/L or less, 30 mg/L or less, 25 mg/L or less, 20 mg/L or less, 15 mg/L or less, 10 mg/L or less, or 5 mg/L or less. The free chlorine concentration therein can be within the range of any combination between these values.

The present disclosure also provides a method, wherein the step of contacting a chlorous acid aqueous solution comprises a step of contacting a microparticulate chlorous acid aqueous solution.

The present disclosure also provides a method, wherein the step of contacting a chlorous acid aqueous solution comprises a step of passing the fowl through a space where a microparticulate chlorous acid aqueous solution is accumulated.

The present disclosure also provides a method, wherein a mean particle size of the microparticulate chlorous acid aqueous solution is 1 um to 10 um. The mean particle size of a chlorous acid aqueous solution can be any of 1 um, 2 um, 3 um, 4 um, 5 um, 6 um, 7 um, 8 um, 9 um, or 10 um, and can be within the range of any combination of these values.

The present disclosure also provides a method, wherein a free chlorine concentration of the chlorous acid aqueous solution is 1 mg/L to 200 mg/L. The free chlorine concentration therein can be 1 mg/L or greater, 5 mg/L or greater, 10 mg/L or greater, 15 mg/L or greater, 20 mg/L or greater, 25 mg/L or greater, 30 mg/L or greater, 35 mg/L or greater, 40 mg/L or greater, 45 mg/L or greater, 50 mg/L or greater, 55 mg/L or greater, 60 mg/L or greater, 65 mg/L or greater, 70 mg/L or greater, 75 mg/L or greater, 80 mg/L or greater, 85 mg/L or greater, 90 mg/L or greater, 95 mg/L or greater, 100 mg/L or greater, 110 mg/L or greater, 120 mg/L or greater, 130 mg/L or greater, 140 mg/L or greater, 150 mg/L or greater, 160 mg/L or greater, 170 mg/L or greater, 180 mg/L or greater, or 190 mg/L or greater. The free chlorine concentration therein can be 200 mg/L or less, 190 mg/L or less, 180 mg/L or less, 170 mg/L or less, 160 mg/L or less, 150 mg/L or less, 140 mg/L or less, 130 mg/L or less, 120 mg/L or less, 110 mg/L or less, 100 mg/L or less, 95 mg/L or less, 90 mg/L or less, 85 mg/L or less, 80 mg/L or less, 75 mg/L or less, 70 mg/L or less, 65 mg/L or less, 60 mg/L or less, 55 mg/L or less, 50 mg/L or less, 45 mg/L or less, 40 mg/L or less, 35 mg/L or less, 30 mg/L or less, 25 mg/L or less, 20 mg/L or less, 15 mg/L or less, 10 mg/L or less, or 5 mg/L or less. The free chlorine concentration therein can be within the range of any combination between these values.

The present disclosure also provides a method, wherein the poultry meat can be used not only as a conventional chicken meat raw material, but also as a raw material of a chicken meat product for raw consumption.

The present disclosure also provides a method, wherein the fowl are a parent stock (spent hen), broiler, roaster, or jidori (free-range chicken).

The present disclosure also provides a method, wherein the method results in enterobacteriaceae adhering to the poultry meat which is 10⁴ cfu/g or less, Campylobacter bacteria which are less than 200/g, Salmonella bacteria which are 0.014 cfu/g or less, and enterohemorrhagic E. coli which is 0.014 cfu/g or less.

The present disclosure also provides a method, wherein the enterobacteriaceae is a group of bacteria revealed based on a testing method according to the ISO methodology (21528).

The present disclosure also provides a method, wherein the Campylobacter bacteria are C. coli, C. concisus, C. curvus, C. fetus, C. gracilis, C. helveticus, C. hominis, C. hyointestinalis, C. insulaenigrae, C. jejuni, C. lanienae, C. lari, C. mucosalis, C. rectus, C. showae, C. sputorum, or C. upsaliensis.

The present disclosure also provides a method, wherein the Salmonella bacteria are S. abortusequi, S. abortusovis, S. typhisuis, S. pullorum, S. gallinarum, S. abortusbovis, S. typhi, S. paratyphi A, S. paratyphi B, S. Bongori, S. typhimurium, S. enteritidis, S. choleraesuis, S. sendai, S. oranienburg, S. chester, or S. arizonae.

The present disclosure also provides a method, wherein the enterohemorrhagic E. coli is E. coli O157, E. coli O26, or E. coli O111.

The present disclosure also provides a method, wherein a step of contacting a chlorous acid aqueous solution is performed in every step of the steps.

One aspect of the present disclosure provides a method of producing poultry meat, comprising:
a step of providing meat of a fowl for consumption; and
a step of contacting the meat of a fowl with a chlorous acid aqueous solution to produce poultry meat. The method can be used for producing poultry meat at a restaurant, etc.

The present disclosure also provides a method, wherein the contact results in Campylobacter bacteria at or below the minimum number of bacteria required for development of a disease (less than 200/g).

The present disclosure also provides a method, wherein the Campylobacter bacteria are C. coli, C. concisus, C. curvus, C. fetus, C. gracilis, C. helveticus, C. hominis, C. hyointestinalis, C. insulaenigrae, C. jejuni, C. lanienae, C. lari, C. mucosalis, C. rectus, C. showae, C. sputorum, or C. upsaliensis.

The present disclosure also provides a method, wherein the poultry meat can be used not only as a conventional chicken meat raw material, but also as a raw material of a chicken meat product for raw consumption.

The present disclosure also provides a method of providing a poultry meat dish comprising a step of preparing a poultry meat dish using poultry meat produced by the method described above.

One aspect of the present disclosure provides a chlorous acid aqueous solution having a mean particle size of 1 um to 10 um. The mean particle size of a chlorous acid aqueous solution can be any of 1 um, 2 um, 3 um, 4 um, 5 um, 6 um, 7 um, 8 um, 9 µm, and 10 um, and can be within the range of any combination of these values.

One aspect of the present disclosure provides a sterilizing agent comprising a microparticulate chlorous acid aqueous solution.

The present disclosure also provides a sterilizing agent, wherein the sterilizing agent is for sterilizing Campylobacter bacteria and enterobacteriaceae.

The present disclosure also provides a sterilizing agent for sterilizing Campylobacter bacteria, wherein the Campylobacter bacteria are C. coli, C. concisus, C. curvus, C. fetus, C. gracilis, C. helveticus, C. hominis, C. hyointestinalis, C. insulaenigrae, C. jejuni, C. lanienae, C. lari, C. mucosalis, C. rectus, C. showae, C. sputorum, or C. upsaliensis.

The present disclosure also provides a sterilizing agent for sterilizing enterobacteriaceae, wherein the enterobacteriaceae is revealed based on a testing method according to the ISO methodology (21528).

One aspect of the present disclosure provides a method of manufacturing a microparticulate chlorous acid aqueous solution by supplying a chlorous acid aqueous solution at an amount of 0.1 L/hour to 10.0 L/hour to a nozzle having a diameter of 5 um to 15 µm, at an amount of air of 10 L/minute to 60 L/minute and at a pressure of 0.04 MPa to 1.2 MPa.

The present disclosure also provides a method, wherein an available concentration of a chlorous acid aqueous solution is 1 mg/L to 50 mg/L. The available chlorine concentration of a chlorous acid aqueous solution can be 1 mg/L or greater, 5 mg/L or greater, 10 mg/L or greater, 15 mg/L or greater, 20 mg/L or greater, 25 mg/L or greater, 30 mg/L or greater, 35 mg/L or greater, 40 mg/L or greater, or 45 mg/L or greater, and can be 50 mg/L or less, 45 mg/L or less, 40 mg/L or less, 35 mg/L or less, 30 mg/L or less, 25 mg/L or less, 20 mg/L or less, 15 mg/L or less, 10 mg/L or less, or 5 mg/L or less. The available chlorine concentration of a chlorous acid aqueous solution can be within the range of any combination between these values.

The present disclosure also provides a method, wherein a spraying time is 2 seconds to 30 seconds.

The present disclosure also provides a method, wherein a mean particle size of a chlorous acid aqueous solution is 8 um or less. The mean particle size of a chlorous acid aqueous solution can be 8 um or less, 7 um or less, 6 um or less, 5 um or less, 4 um or less, 3 um or less, 2 um or less, or 1 um or less.

The present disclosure also provides a method, wherein a shape of a microparticle of the chlorous acid aqueous solution is round, oval, diamond, or omnidirectional.

The present disclosure also provides a method, wherein a spraying speed is 0.39 L/minute to 3.2 L/minute.

The present disclosure also provides a method, wherein a spraying speed is 0.9 L/hour to 2.0 L/hour.

One aspect of the present disclosure provides an apparatus for manufacturing a microparticulate chlorous acid aqueous solution, comprising a nozzle having a diameter of 1 um to 10 um and means for providing a chlorous acid aqueous solution at an amount of 0.1 L/hour to 10.0 L/hour, at an amount of air of 10 L/minute to 60 L/minute and at a pressure of 0.04 MPa to 1.2 MPa.

The present disclosure also provides an apparatus, wherein an available concentration of a chlorous acid aqueous solution is 1 mg/L to 50 mg/L. The available chlorine concentration of a chlorous acid aqueous solution can be 1 mg/L or greater, 5 mg/L or greater, 10 mg/L or greater, 15 mg/L or greater, 20 mg/L or greater, 25 mg/L or greater, 30 mg/L or greater, 35 mg/L or greater, 40 mg/L or greater, or 45 mg/L or greater, and can be 50 mg/L or less, 45 mg/L or less, 40 mg/L or less, 35 mg/L or less, 30 mg/L or less, 25 mg/L or less, 20 mg/L or less, 15 mg/L or less, 10 mg/L or less, or 5 mg/L or less. The available chlorine concentration of a chlorous acid aqueous solution can be within the range of any combination between these values.

The present disclosure also provides an apparatus, wherein a spraying time is 2 seconds to 30 seconds.

The present disclosure also provides an apparatus, wherein a mean particle size of a chlorous acid aqueous solution is 8 um or less. The means particle size of a chlorous acid aqueous solution can be 8 um or less, 7 um or less, 6 um or less, 5 um or less, 4 um or less, 3 um or less, 2 um or less, or 1 um or less.

The present disclosure also provides an apparatus, wherein a shape of a microparticle of the chlorous acid aqueous solution is round, oval, diamond, or omnidirectional.

The present disclosure also provides an apparatus, wherein a spraying speed is 0.39 L/minute to 3.2 L/minute.

The present disclosure also provides an apparatus, wherein a spraying speed is 0.9 L/hour to 2.0 L/hour.

One aspect of the present disclosure provides a system for sterilizing meat of a fowl for consumption, comprising:
a spray nozzle for spraying a chlorous acid aqueous solution;
an instrument for providing the meat of a fowl;
means for moving the instrument; and
a barrier for forming a processing space for blocking a side surface with respect to a direction of movement of the instrument.

The present disclosure also provides a system further comprising a spraying zone for spraying a chlorous acid aqueous solution.

The present disclosure also provides a system, wherein the spray nozzle provides a chlorous acid aqueous solution having a mean particle size of 1 um to 10 um. The mean particle size of a chlorous acid aqueous solution can be any of 1 um, 2 um, 3 um, 4 um, 5 um, 6 um, 7 um, 8 um, 9 um, or 10 um, and can be within the range of any combination of these values.

The present disclosure also provides a system, wherein the sterilization of the meat of a fowl is sterilization of a chicken meat raw material that can be used not only as a conventional chicken meat raw material, but also as a raw material of a chicken meat product for raw consumption.

One aspect of the present disclosure provides a system, based on HACCP, for manufacturing poultry meat, comprising: at least one unit selected from the group consisting of a blood-letting unit, a scalding unit, a defeathering unit, an evisceration unit, an inside/outside cleaning unit, a cooling unit, and a refrigeration unit; and a unit or means for contacting a chlorous acid aqueous solution.

The present disclosure also provides a system, wherein the unit for contacting a chlorous acid aqueous solution comprises a unit for spraying a chlorous acid aqueous solution.

The present disclosure also provides a system, wherein the unit for contacting a chlorous acid aqueous solution comprises a unit comprising a chlorous acid aqueous solution supplemented solution.

The present disclosure also provides a system, wherein cleaning water used in the inside/outside cleaning unit comprises a chlorous acid aqueous solution.

The present disclosure also provides a system, wherein cooling water used in the cooling unit comprises a chlorous acid aqueous solution.

The present disclosure also provides a system for a chicken meat raw material, wherein the poultry meat can be used not only as a conventional chicken meat raw material, but also as a raw material of a chicken meat product for raw consumption.

The present disclosure also provides use of a chlorous acid aqueous solution for the manufacture of poultry meat.

The present disclosure also provides use of a chlorous acid aqueous solution for the manufacture of poultry meat that can be consumed raw.

The present disclosure also provides use of a chlorous acid aqueous solution for sterilizing poultry meat.

### (Flow of poultry processing steps)

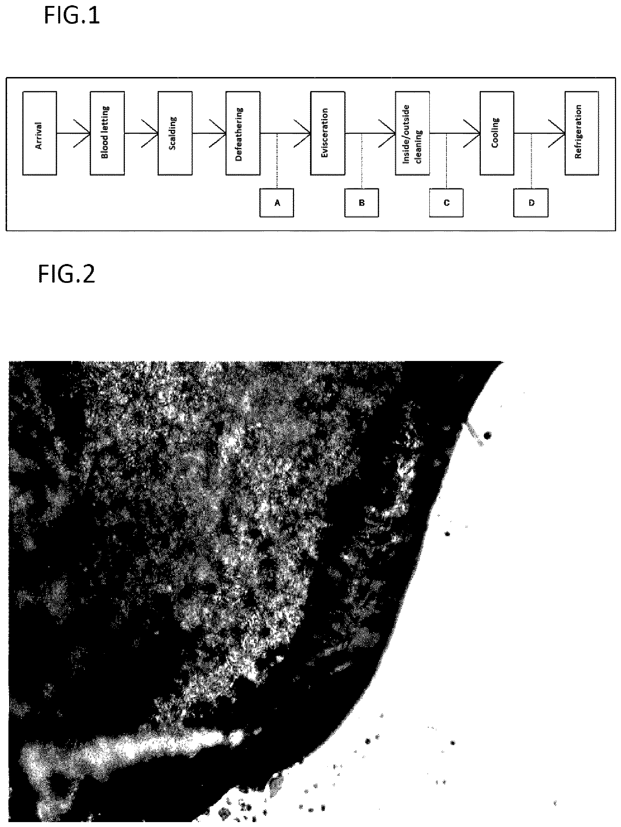

The poultry processing flow of the present disclosure is described with reference to Figure 1. Generally, purchased a live fowl can be placed in a cage, stacked onto the bed of a truck, and transported. Feces excreted by a chicken in a cage above falls to the cage below during transport, so that the live chicken in the cage below can be covered with feces. Since chickens are exposed to blazing sun during the summer, cold water is sprinkled from the top of the cage. Thus, the cage at the very bottom is the most contaminated, and can emit a fermented stuffy odor. Moreover, shackling starts with a live fowl in the bottom cage, so that chickens can enter a line from the most contaminated chicken.

Live shackled chickens are slaughtered in a slaughtering room, and allowed to bleed out in a blood-letting unit. Chickens without blood are scalded in hot water in a scalding unit and cleaned with warm water. The carcass after scalding is defeathered in a defeathering unit and singed. The defeathered carcass is sterilized by apparatus A, which sprays microparticles (mist) of a chlorous acid aqueous solution.

The carcass sterilized by apparatus A is eviscerated by removing the organs in an evisceration unit. The eviscerated carcass is sterilized by apparatus B, which sprays microparticles (mist) of a chlorous acid aqueous solution. The carcass sterilized by apparatus B is cleaned by an inside/outside cleaning unit. Inside/outside cleaning water for cleaning inside/outside can comprise a chlorous acid aqueous solution. The free chlorine concentration of inside/outside cleaning water supplemented with a chlorous acid aqueous solution can be 1 to 100 mg/L. Inside/outside cleaning water comprising a chlorous acid aqueous solution can also sterilize microorganisms derived from an organ while rinsing off organ content adhering to the surface of the carcass. The carcass after inside/outside cleaning is sterilized by apparatus C, which sprays microparticles (mist) of a chlorous acid aqueous solution.

The carcass sterilized by apparatus C is cooled in a cooling unit. Cooling water used in a cooling unit can comprise a chlorous acid aqueous solution. The free chlorine concentration of cooling water supplemented with a chlorous acid aqueous solution can be 1 to 100 mg/L. Cooling water comprising a chlorous acid aqueous solution can sterilize microorganisms/bacteria adhering to the surface of the chicken body while cooling.

A cooling unit can comprise a pre-cooling chiller tank and a main cooling chiller tank. A pre-cooling chiller tank can remove heat of a carcass and reduce the heat to a certain value, and a main cooling chiller tank can further cool the carcass cooled by pre-cooling.

The carcass cooled in the cooling unit can be sterilized by apparatus D, which sprays microparticles (mist) of a chlorous acid aqueous solution, and then stored in a refrigeration unit. The carcass in the refrigeration unit can be shipped in response to an order.

### (Apparatus which sprays microparticles (mist) of a chlorous acid aqueous solution)

The present disclosure discloses an apparatus, which sprays a chlorous acid aqueous solution as microparticles (mist) in order to contact the chlorous acid aqueous solution efficiently with the carcass of a chicken. While chlorous acid aqueous solutions can sterilize a carcass of a chicken by scalding, scalding uses a large amount of chlorous acid aqueous solution and is thus costly. The present disclosure can efficiently contact a chlorous acid aqueous solution with a carcass by using a chlorous acid aqueous solution as microparticles (mist).

A chlorous acid aqueous solution can be modified by the spraying apparatus of the present disclosure to have a mean particle size which is small to the extent that a hand would not be wet upon contact (e.g., 1 um to 10 µm). Since a chlorous acid aqueous solution is a relatively stable substance, it can be readily converted into microparticles (mist). Microparticles (mist) can be produced by supplying a chlorous acid aqueous solution at an amount of 0.1 L/hour to 10.0 L/hour to a nozzle having a diameter of 5 um to 15 µm, at an amount of air of 10 L/minute to 60 L/minute and at a pressure of 0.04 MPa to 1.2 MPa.

A chlorous acid aqueous solution mist-producing and spraying apparatus can have the following properties.
The available concentration of a chlorous acid aqueous solution: 25 mg/L or less
Spraying time: 2 seconds to 30 seconds
Mist spraying apparatus: the chlorous acid aqueous solution is converted into microparticles that are 8 um or less.

The spray shape of microparticles can be round, oval, diamond, or omnidirectional.

The pump pressure can be 0.04 to 1.2 MPa. The spray speed can be 0.39 L/min to 3.2 L/min (high pressure), or 0.9 L/h to 2.0 L/h (low pressure).

A chlorous acid aqueous solution converted into microparticles (mist) by a spraying apparatus can be accumulated at a specific location within a poultry processing factory. A chlorous acid aqueous solution can be contacted with a carcass by passing the carcass through a space where the microparticles (mist) of the chlorous acid aqueous solution are accumulated.

A microparticulate (mist) chlorous acid aqueous solution can permeate through chicken skin surrounded by oil to exert a high sterilization effect (Figure 2). A sterilization method using such a microparticulate (mist) chlorous acid aqueous solution can dramatically reduce the cost and the amount of sterilizing agent used.

In comparison to conventional scalding disinfection methods requiring a large scalding tank, a mist spraying apparatus can be installed in a part of a space in a chicken disassembly line and can be readily introduced into a poultry processing line.

### (Test for studying the effect of reducing Campylobacter contamination by a chlorous acid aqueous solution at a poultry processing factory)

Reduction in the number of Campylobacter on the chicken carcass surface due to misting was investigated. The number is reduced to 50 Campylobacter bacteria /g or less. In the investigative test, the shape of a spray space was height (0.5 m to 3 m) × width (0.5 m to 2 m) × length (1 m to 5 m).

### (Establishment of a Campylobacter sterilization system that is effective throughout the year)

According to the National Institute of Health Sciences, the minimum number of bacteria required for development of a Campylobacter disease is estimated to be 500 to 800 CFU. It is also known that contamination of chicken due to Campylobacter varies by season. In this regard, the status of Campylobacter contamination of chicken carcasses throughout the year at a large-scale poultry processing factory was investigated, and the effect of reducing Campylobacter with the operation of the sterilization system described above was investigated by determining the bacteria count. The cost of operating the apparatus described above was calculated to study the materializable cost- effectiveness.

### (Ripple effect expected from the present disclosure)

While there is no specific indicator for economic loss from food poisoning due to Campylobacter, etc. in Japan, this is assessed by the "expense required for testing and treatment of diseases" and "economic loss from missing work due to illness" in the US and Europe. The economic loss due to Campylobacter in Japan was calculated by assuming that the ratio of food poisoning from chicken meat to the population in each country exhibits the same tendency.

**[Table 1]**

| Item | Maximum value (million yen) | Minimum value (million yen) |
|---|---|---|
| Expense required for testing and treatment of diseases | 1792 | 1081 |
| Economic loss from missing work due to illness | 6311 | 5172 |
| Total | 8103 | 6253 |

Reference documents: Prevention of Poultry-Borne Salmonellosis by Irradiation, Costs and Benefits in Scotland W. J. Reilly, B. F. Yule, G. I. Forbes, and J. C. M. Sharp Ruchill Hospital, Glasgow, Scotland, United Kingdom IAEA-SM-328/67
Reference documents: Social Cost of Foodborne Disease, Ushio Shimizu, Jpn. j. Food Microbiol. 19(3). 87-94. 2002

If Campylobacter can be controlled by the method of the present disclosure, safety of branded chicken meat and chicken meat processed products in the southern Kyushu region would be ensured, so that an economic ripple effect of 6.2 billion to 8.1 billion yen can be expected just in Japan when economic loss from Campylobacter in Japan is estimated higher as shown in the table described above.

### (Chlorous acid aqueous solution and manufacturing example thereof)

The chlorous acid aqueous solution used in the present disclosure has the features found by the inventors. A chlorous acid aqueous solution manufactured by any method, such as a known manufacturing method described in a reference described above, can be used. Examples of the typical composition that can be used include, but are not limited to, a combination of 61.40% chlorous acid aqueous solution, 1.00% potassium dihydrogen phosphate, 0.10% potassium hydroxide, and 37.50% purified water (sold by the Applicant; 72% chlorous acid aqueous solution corresponds to 30000 ppm of chlorous acid). This agent reduces the decrease in chlorous acid due to contact with an organic matter under an acidic condition, but maintains the sterilization effect. The agent also has a feature of having minor chlorine gas generation, which suppresses amplification of odor from the mixture of chlorine and organic matter.

In one embodiment, the chlorous acid aqueous solution of the present disclosure can be produced by adding and reacting sulfuric acid or an aqueous solution thereof to an aqueous sodium chlorate solution at an amount and concentration where the pH value of the aqueous solution can be maintained at 3.4 or lower to generate chloric acid, and subsequently adding hydrogen peroxide in an amount equivalent to or greater than the amount required for a reduction reaction of the chloric acid.

Further, in another embodiment, the chlorous acid aqueous solution of the present disclosure can be produced by adding one inorganic acid or inorganic acid salt, two or more types thereof, or a combination thereof to an aqueous solution prepared from producing chlorous acid by adding and reacting sulfuric acid or an aqueous solution thereof to an aqueous sodium chlorate solution at an amount and concentration where the pH value of the aqueous solution can be maintained at 3.4 or lower to generate chloric acid, and subsequently adding hydrogen peroxide in an amount equivalent to or greater than the amount required for a reduction reaction of the chloric acid, and adjusting the pH value within the range from 3.2 to 8.5.

Furthermore, in another embodiment, the chlorous acid aqueous solution of the present disclosure can be produced by adding one inorganic acid, inorganic acid salt, organic acid, or organic acid salt, two or more types thereof, or a combination thereof to an aqueous solution prepared from producing chlorous acid by adding and reacting sulfuric acid or an aqueous solution thereof to an aqueous sodium chlorate solution at an amount and concentration where the pH value of the aqueous solution can be maintained at 3.4 or lower to generate chloric acid, and subsequently adding hydrogen peroxide in an amount equivalent to or greater than the amount required for a reduction reaction of the chloric acid, and adjusting the pH value within the range from 2.9 to 8.5. Further still, in another embodiment, the chlorous acid aqueous solution of the present disclosure can be produced by adding one inorganic acid, inorganic acid salt, organic acid, or organic acid salt, two or more types thereof, or a combination thereof after adding one inorganic acid or inorganic acid salt, two or more types thereof, or a combination thereof to an aqueous solution prepared from producing chlorous acid by adding and reacting sulfuric acid or an aqueous solution thereof to an aqueous sodium chlorate solution at an amount and concentration where the pH value of the aqueous solution can be maintained at 3.4 or lower to generate chloric acid, and subsequently adding hydrogen peroxide in an amount equivalent to or greater than the amount required for a reduction reaction of the chloric acid, and adjusting the pH value within the range from 3.2 to 8.5.

Further, in another embodiment, carbonic acid, phosphoric acid, boric acid, or sulfuric acid can be used as the inorganic acid in the method described above.

Further still, in another embodiment, carbonate, inorganic hydroxide, phosphate, or borate can be used as the inorganic acid salt.

Further, in another embodiment, sodium carbonate, potassium carbonate, sodium bicarbonate, or potassium bicarbonate can be used as the carbonate.

Furthermore, in another embodiment, sodium hydroxide, potassium hydroxide, calcium hydroxide, or barium hydroxide can be used as the inorganic hydroxide.

Further still, in another embodiment, disodium hydrogen phosphate, sodium dihydrogen phosphate, trisodium phosphate, tripotassium phosphate, dipotassium hydrogen phosphate, or potassium dihydrogen phosphate can be used as the phosphate.

Further, in another embodiment, sodium borate or potassium borate can be used as the borate.

Furthermore, in another embodiment, succinic acid, citric acid, malic acid, acetic acid, or lactic acid can be used as the organic acid.

Further still, in another embodiment, sodium succinate, potassium succinate, sodium citrate, potassium citrate, sodium malate, potassium malate, sodium acetate, potassium acetate, sodium lactate, potassium lactate, or calcium lactate can be used as the organic acid salt.

In a method of manufacturing an aqueous solution comprising chlorous acid (HClO₂) (chlorous acid aqueous solution) that can be used as a bacteria-killing agent, chlorous acid (HClO₂) is produced by adding hydrogen peroxide (H₂O₂) in an amount required to produce chlorous acid by a reducing reaction of chloric acid (HClO₃) obtained by adding sulfuric acid (H₂SO₄) or an aqueous solution thereof to an aqueous solution of sodium chlorate (NaClOs) so that the aqueous solution is in an acidic condition. The basic chemical reaction of this method of manufacturing is represented by the following formula A and formula B.
[Chemical Formula 1]

**2NaClO₃+H₂SO₄ → 2HClO₃+Na₂SO₄ ↓** **(formula A)**

**HClO₃+H₂O₂ → HClO₂+H₂O+O₂** ↑ **(formula B)**

Formula A indicates that chloric acid is obtained by adding sulfuric acid (H₂SO₄) or an aqueous solution thereof at an amount and concentration where the pH value of an aqueous sodium chlorate (NaClOs) solution can remain acidic. Next, formula B indicates that chloric acid (HClO₃) is reduced by hydrogen peroxide (H₂O₂) to produce chlorous acid (HClO₂).
[Chemical Formula 2]

HClO₃+H₂O₂ → 2 ClO₂+H₂O+O₂↑ (formula C)

2ClO₂+H₂O₂ → 2 HClO₂+O₂↑ (formula D)

2 ClO₂+H₂O ⇔ HClO₂+HClO₃ (formula E)

2 HClO₂⇔H₂O+Cl₂O₃ (formula F)

At this time, chlorine dioxide gas (ClO₂) is generated (formula C). However, coexistence with hydrogen peroxide (H₂O₂) produces chlorous acid (HClO₂) through the reactions in formulas D to F.

Meanwhile, the produced chlorous acid (HClO₂) has the property of being decomposed early into chlorine dioxide gas or chlorine gas due to the presence of chloride ion (Cl⁻), hypochlorous acid (HClO), and other reduction product, or resulting in a plurality of chlorous acid molecules reacting to decompose one another. Thus, it is necessary to prepare chlorous acid (HClO₂) so that the state of chlorous acid can be sustained for an extended period of time in order to be useful as a bacteria-killing agent.

In this regard, chlorous acid (HClO₂) can be stably sustained over an extended period of time from creating a transition state to delay a decomposition reaction by adding one inorganic acid, inorganic acid salt, organic acid, or organic acid salt, two or more types thereof, or a combination thereof to the chlorous acid (HClO₂) or chlorine dioxide gas (ClO₂) obtained by the method described above or an aqueous solution containing them.

In one embodiment, it is possible to utilize a mixture prepared by adding an inorganic acid or inorganic acid salt, specifically carbonate or inorganic hydroxide, two or more types thereof, or a combination thereof to the chlorous acid (HClO₂) or chlorine dioxide gas (ClO₂) obtained by the method described above or an aqueous solution containing them.

In another embodiment, it is possible to utilize a mixture prepared from adding one inorganic acid, inorganic acid salt, organic acid, or organic acid salt, two or more types thereof, or a combination thereof to an aqueous solution to which inorganic acid or inorganic acid salt, specifically carbonate or inorganic hydroxide, two or more types thereof, or a combination thereof is added.

Additionally, in another embodiment, it is possible to utilize a mixture prepared by adding one inorganic acid, inorganic acid salt, organic acid, or organic acid salt, two or more types thereof, or a combination thereof to an aqueous solution manufactured by the method described above.

Examples of the inorganic acid described above include carbonic acid, phosphoric acid, boric acid, and sulfuric acid. Examples of the inorganic acid salt include phosphate and borate, in addition to carbonate and inorganic hydroxide. Specifically, sodium carbonate, potassium carbonate, sodium bicarbonate, or potassium bicarbonate may be used as the carbonate; sodium hydroxide, potassium hydroxide, calcium hydroxide, or barium hydroxide may be used as the inorganic hydroxide; disodium hydrogen phosphate, sodium dihydrogen phosphate, trisodium phosphate, tripotassium phosphate, dipotassium hydrogen phosphate, or potassium dihydrogen phosphate may be used as the phosphate; and sodium borate or potassium borate may be used as the borate. Examples of the organic acid described above include succinic acid, citric acid, malic acid, acetic acid, and lactic acid. Further, sodium succinate, potassium succinate, sodium citrate, potassium citrate, sodium malate, potassium malate, sodium acetate, potassium acetate, sodium lactate, potassium lactate, or calcium lactate is suitable as the organic acid salt.

When an acid and/or a salt thereof is added, a transition state such as Na⁺ + ClO₂⁻ < - > Na⁻ClO₂, K⁺ + ClO₂⁻ < - > K-ClO₂, or H⁺ + ClO₂⁻ < - > H-ClO₂ can be temporarily created to delay the progression of chlorous acid (HClO₂) to chlorine dioxide (ClO₂), which enables the manufacture of an aqueous solution comprising chlorous acid (HClO₂) that sustains chlorous acid (HClO₂) for an extended period of time and generates a low amount of chlorine dioxide (ClO₂).

The following represents the decomposition of chlorite in an acidic solution.
[Chemical Formula 3]

5ClO₂⁻+4H⁺→4ClO₂+5Cl⁻+2H₂O (a)

(5NaClO₂+4CH₃COOH →4ClO₂+4CH₃COONa+NaCl+2H₂O) 3ClO₂⁻→2ClO₃⁻+Cl⁻ (b)

(3NaClO₂→2NaClO₃+NaCl) autodegradation ClO₂**⁻**→Cl⁻+2O (c)

As expressed in the formula, the rate of decomposition of an aqueous chlorite solution is greater at lower pH, i.e., more acidic. Specifically, the absolute rates of reactions (a), (b), and (c) in the formula described above increase. For example, although the ratio accounted for by reaction (a) decreases at a lower pH, the total decomposition ratio changes significantly, i.e., to a larger value. Thus, the amount of generated chlorine dioxide (ClO₂) also increases as the pH decreases. Therefore, a lower pH value results in sooner sterilization or bleaching. However, irritable and harmful chlorine dioxide gas (ClO₂) renders an operation more difficult and adversely affects the health of a human being. Further, a reaction of chlorous acid to chlorine dioxide progresses quickly to render chlorous acid unstable. In addition, the period during which a sterilizing power can be sustained is very short.

When the inorganic acids, inorganic acid salts, organic acids, or organic acid salts described above are added to an aqueous solution comprising chlorous acid (HClO₂), pH values are adjusted in the range of 2.9 to 8.5 from the viewpoint of balancing suppression of the generation of chlorine dioxide and sterilizing power.

The chlorous acid aqueous solution of the present disclosure can also be an aqueous solution obtained by adding sulfuric acid to an aqueous solution obtained by electrolysis of a solution prepared by adding hydrochloric acid to a saturated sodium chloride solution under acidic conditions in a diaphragm-free electrolytic tank (referred to those comprised of anode and cathode that are not separated by a diaphragm) to make the aqueous solution strongly acidic, and adding a hydrogen peroxide solution for a reaction with chloric acid generated thereby. A chlorous acid aqueous solution can be those described in Japan's Specifications and Standards for Food Additives, 9th Edition 2018 (Ministry of Health, Labour and Welfare; Consumer Affairs Agency).

Examples of chlorous acid aqueous solution formulations that can be used in the present disclosure include HonbuSankei's "Kare for Hands", "Rare for Hands Pro-free", "Rare for Fresh", "Autoloc Super", "New Autoloc SP", "Rare for peace Pro-free", "Rare for No. 3", "Rare for Norobarrier Plus", "KlorusKare 8", "KlorusKare 10", etc.

### (Problems in comparing/evaluating antimicrobial effects of chlorous acid aqueous solutions and sodium hypochlorite)

Comparison/evaluation of antimicrobial effects of chlorous acid aqueous solutions and sodium hypochlorite is problematic in that the concentration of chlorine oxide can be denoted in terms of available chlorine concentration or free chlorine, while antimicrobial effects are dependent on free chlorine, which is the source of oxidation power. In addition, sodium hypochlorite has a near 1:1 relationship between free chlorine and available chlorine concentrations, but the available chlorine concentration and free chlorine do not necessarily match for chlorous acid aqueous solutions in the same manner as sodium hypochlorite. For this reason, it is necessary to compare sterilizing powers of both agents on a level field by using oxidation power that represents the antimicrobial effect, i.e., free chlorine, instead of available chlorine concentrations.

Oxidation power of a chlorine oxide agent is generally found through a measurement method utilizing colorimetry such as a TMB method or DPD method. However, there is no standard for measuring free chlorine in a chlorous acid aqueous solution in the same manner as sodium hypochlorite. For this reason, calibration curves are created by using 1 mg/L of free chlorine (as Cl) of sodium hypochlorite as 1 oxidation power. Oxidation power can be represented by free chlorine (as Cl). For comparison at the same free chlorine level, free chlorine (as Cl) of sodium hypochlorite is generated from Cl radicals, but free chlorine of chlorous acid aqueous solutions has HClO₂ as the source of generation. Thus, it is difficult to compare when evaluating with respect to a standard in accordance therewith. Therefore, a chlorous acid aqueous solution is compared and evaluated with sodium hypochlorite on a level field by using the same standard through calculation at oxidation power 1 = free chlorine (as Cl) of 1 mg/L in the same manner as sodium hypochlorite.

As a measuring method of free chlorine (as Cl), a buffer and a DPD indicator are added to a sample and a wavelength of 510 nm is measured with an absorption spectrophotometer, and for measurement of free chlorine (as Cl) in the presence of an organic matter, measurement is taken at a wavelength of 655 nm using a TMB reagent to deduce the concentration from the measurement values. Further, a method of conducting a test for studying the sterilization effect includes preparing free chlorine (as Cl) of a test agent by a DPD method, contacting each agent with an organic matter-containing bacterial solution, neutralizing the solution with sodium thiosulfate after a certain period of time has passed, and checking the viable bacteria count in the neutral solution.

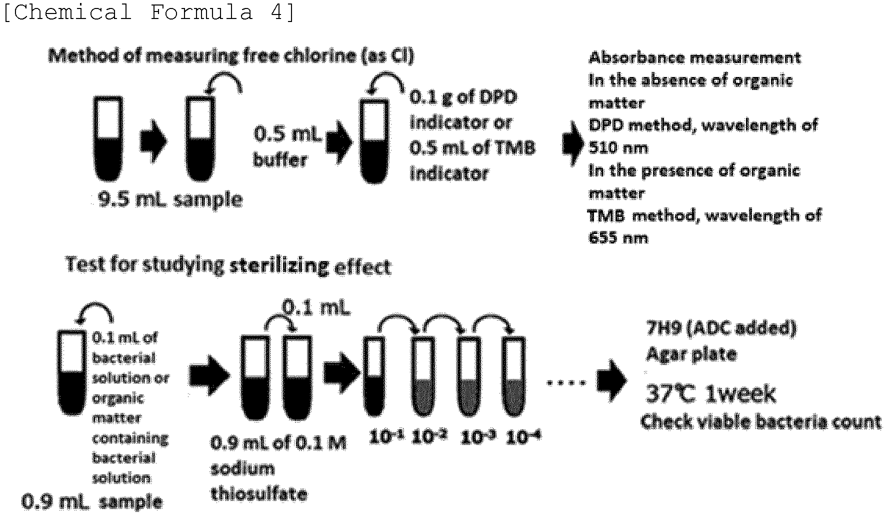

### (Test for checking chlorous acid aqueous solution permeation)

A carcass of chicken meat is surrounded by oil and fat of the chicken skin. When sprayed onto the carcass by a normal spraying method, the carcass surface would be in a "wet" state, and oil and fat of the chicken skin would repel water. Thus, it is known that a sterilizing agent would not reach Campylobacter that is deep in the follicles on the chicken skin and is thus unable to sterilize. In this regard, a test was conducted to check whether a sterilizing agent permeates to the deep part of follicles by preparing microparticles which do not wet a hand upon contact, even on chicken skin surrounded by oil.

Inkjet ink (cyan) is converted into microparticles that are 8 um or less. 5 cm × 5 cm chicken skin was immersed in a space filled with the microparticles in a plastic case, left standing for one minute, and then sliced and observed through an optical microscope. A picture is shown in Figure 2. The picture shows that the inkjet ink has contacted and permeated through the chicken skin.

### (Superiority of chlorous acid aqueous solution)

While chlorous acid aqueous solutions have the same sterilization effect as acidified sodium chlorite (ASC) and hypochlorous acid water in terms of in vitro sterilizing power, chlorous acid aqueous solutions and alcohol have a better sterilization effect on Campylobacter on a carcass (in vivo). Safety of workers needs to be considered on poultry processing lines. Chlorous acid aqueous solutions, which are tasteless, odorless, harmless, non-stimulatory, and nonflammable, are the safest. Chlorous acid aqueous solutions also have no effect on a final product. Although cost can be an issue, the cost can be sufficiently within an economic range by the method of the present disclosure.

In this regard, diluents of chlorous acid aqueous solution, sodium hypochlorite, sodium chlorite, peracetic acid formulation, sodium hypochlorite, and alcohol adjusted to each of the concentrations were loaded into the spraying apparatus used in Example 1-1 to investigate the sterilization effect on an eviscerated carcass inoculated with E. coli and the minimum concentration at which an effect was manifested. The state of the eviscerated carcass at the time was evaluated by workers working on site.

**[Table 2]**

| Compared items | Chlorous acid aqueous solution | Sodium hypochlorite | Sodium chlorite | Peracetic acid formulation | Hypochlorous acid water | Alcohol |
|---|---|---|---|---|---|---|
| Sterilization power (in vitro) | ⊚ | Δ | ⊚ | Δ | ⊚ | Δ |
| | 5mg/L | 50mg/L | 5mg/L | 200mg/L | 5mg/L | 80%(v/v) or greater |
| Sterilization power (in vivo) | ⊚ | × | × | × | × | ⊚ |
| | 20mg/L | | 1200mg/L (undiluted solution) | 2000mg/L (undiluted solution) | 80mg/L (undiluted solution) | 100%(v/v) (undiluted solution) |
| Safety | ⊚ | × | × | × | ○ | × |
| Quality to product | ○ | × | ○ | × | ○ | × |
| Operability | ○ | ○ | × | × | ○ | × |
| Cost | Δ | ⊚ | Δ | Δ | × | ○ |

Hypochlorous acid water had a low free chlorine concentration obtained as electrolyzed water, and lost a sterilization effect in the presence of protein or fat of chicken skin.

Sodium hypochlorite not only loses a sterilization effect in the presence of protein or oil of chicken skin, but also generates chlorine gas when converted into microparticles, so that sodium hypochlorite was evaluated as unable to ensure safety of workers.

The primary component of ASC is chlorous acid and has a sterilization effect on Campylobacter bacteria in the presence of an organic matter. Meanwhile, due to preparation upon use, unbalancing reactions actively occur to generate a large amount of chlorine dioxide gas upon formation of microparticles. Thus, ASC was evaluated as unable to ensure safety of workers.

It is reported that peracetic acid can have a sterilization effect on Campylobacter bacteria even in the presence of an organic matter. However, a sterilization effect was not found when used after dilution to the extent that eliminated acetic acid odor. Further, due to the hydrogen peroxide content, peracetic acid needs to be used with consideration for the effect on the factory building. When peracetic acid was converted into microparticles and sprayed in a building at a concentration exhibiting efficacy, acetic acid odor was found, and workers complained about the odor.

Alcohol can degrade oil and contact with Campylobacter bacteria. For this reason, the highest sterilization effect was observed. However, commercial value as "tataki" was lost at a concentration where an effect was observed. Moreover, due to the flammability, alcohol was evaluated as unable to ensure safety of workers.

Chlorous acid aqueous solutions are tasteless and odorless, and workers did not file any complaints. An effect on wastewater was not found. Rather, chlorous acid aqueous solutions improved the ability to clean wastewater. Further, workers did not complain of safety concerns. In view of the above, chlorous acid aqueous solutions are deemed the only sterilizing agent that can be used in practice.

Apparatuses that can maintain a semi-sealed space filled with a chlorous acid aqueous solution formulation (microparticles (diameter of 8 um or less) with a free chlorine concentration of 25 mg/L (as Cl = 35.45)) were placed at three locations, i.e., "after a defeathering step and before an evisceration step", "after an evisceration step and before an inside/outside cleaning step", and "after an inside/outside cleaning step and before a cooling step". Broilers were passed therethrough at a line speed of about 50 broilers/minute. Broilers collected in each step were thoroughly skinned and used as samples. Campylobacter bacteria were checked using a quantification method (Butzler's medium or mCCDA medium of SEL formulation) and the MPN method (method using three sets and three tubes). Enterobacteriaceae were checked in accordance with ISO 21528-1.

Broilers contaminated with feces during the distribution process are contaminated with Campylobacter bacteria derived from a farm. In this regard, when a semi-sealed space filled with microparticles of a chlorous acid aqueous solution was provided "after a defeathering step and before an evisceration step" during an actual process in a poultry processing factory and a defeathered carcass was passed therethrough, an effect of reducing Campylobacter bacteria derived from a farm was observed. Contamination derived from the intestinal tract (organ content) then occurs via an evisceration step so that the number of Campylobacter bacteria reverts back to the number before the defeathering step. Meanwhile, when a semi-sealed space filled with microparticles of a chlorous acid aqueous solution is provided "after an evisceration step and before an inside/outside cleaning step" and "after an inside/outside cleaning step and before a cooling step" and then the eviscerated carcass is passed therethrough, an effect of reducing Campylobacter bacteria derived from the intestinal tract contained in the organ content was observed. It was found in view of the above that secondary contamination which has occurred in the evisceration step can be reduced to the state before the evisceration step by chemical sterilization treatment using a chlorous acid aqueous solution. Furthermore, Campylobacter bacteria on the eviscerated carcass that have passed through a pre-cooling tank and a main cooling tank were reduced to 10¹ CFU/10 g or less, so that the contamination was successfully reduced to a state where food poisoning due to Campylobacter bacteria can be prevented. Change in Campylobacter bacteria and enterobacteriaceae contamination reduced by spraying microparticles of a chlorous acid aqueous solution was observed to have a tendency to decrease after the defeathering step and then increase after the evisceration step, and decrease again after inside/outside cleaning, and decrease to a state where meat can be consumed raw after the cooling step throughout the year. Meanwhile, a tendency of the contamination spreading to the carcass surface after evisceration was observed for enterobacteriaceae. However, it was found that severe contamination in the eviscerated carcass can be dramatically reduced by chemical sterilization treatment using a chlorous acid aqueous solution. These results confirmed how a chemical sterilization treatment using a chlorous acid aqueous solution can be effective means for measures against infections and measures for preventing food poisoning in actual practice. As a result, there is a prospect for securing raw materials of dishes for raw consumption that does not lead to a food poisoning incident. Therefore, it is proposed that establishment of specifications for poultry meat for raw consumption as a raw material of dishes for raw consumption is essential in order to protect the raw consumption culture unique to Japan.

Reference literature such as scientific literature, patents, and patent applications cited herein are incorporated herein by reference to the same extent that the entirety of each document is specifically described.

As described above, the present disclosure has been described while showing preferred embodiments to facilitate understanding. While the present disclosure is described hereinafter based on Examples, the above descriptions and the following Examples are not provided to limit the present invention, but for the sole purpose of exemplification. Thus, the scope of the present invention is not limited to the embodiments and Examples specifically described herein and is limited only by the scope of claims.

### [Examples]

### (Quantification method of chlorous acid aqueous solution)

About 5 g of the present product is precisely measured. Water is added thereto so that the solution is exactly 500 ml to prepare a sample solution. After 20 ml of the sample solution is accurately measured, placed in an iodine flask, and combined with 10 ml of sulfuric acid (1_{→}10), 1 g of potassium iodide is added thereto. The flask is immediately plugged and shaken well. 5 ml of a potassium iodide reagent is poured into the top portion of the iodine flask and left standing in the dark for 15 minutes. The plug is then loosened to pour in a potassium iodide reagent and the flask is plugged immediately. After thoroughly mixing, freed iodine is titrated with 0.1 mol/L sodium thiosulfate (indicator: 5 ml of starch reagent), wherein the starch reagent is added when the color of the solution has changed to a light yellow color near the end point, where the endpoint is when blue color of the solution disappears. A blank test is separately conducted for correction (1 mL of 0.1 mol/L sodium thiosulfate solution = 1.711 mg of HClO₂).

### (Manufacturing Examples)

Chlorous acid aqueous solution formulations used in the following Examples were manufactured as follows. Chlorous acid aqueous solution may be abbreviated as "CAAS" herein, but the terms are synonymous.

Component analysis table for chlorous acid aqueous solution

**[Table 3]**

| CAAS specification | Specification Value | Match/Not a Match |
|---|---|---|
| Content | 4-6 % | 4.1% |
| Attribute | Light yellowish green to yellowish red | yellowish red |
| Confirmation Test (1) | When 0.1 ml of potassium permanganate solution (1→300) is added to 5 ml of an aqueous solution of the present product (1→20), the solution turns reddish purple. When 1 ml of sulfuric acid (1→20) is added thereto, the solution turns light yellow. | Match |
| Confirmation Test (2) | An aqueous solution of the present product (1→20) has maximum absorbance sections at wavelengths 258 to 262 nm and 346 to 361 nm. | Match |
| Confirmation Test (3) | If potassium iodide starch paper is dipped in the present product, the potassium iodide starch paper changes to a blue color and then the color fades. | Match |
| Purity Test (1) | 1.0 µg/g or lower for lead | Match |
| Purity Test (2) | 1.0 µg/g or lower for Ag₂O₃ | Match |

A chlorous acid aqueous solution formulation was manufactured using this chlorous acid aqueous solution based on the following composition.

**[Table 4]**

| | Raw material name | Blended amount | Concentration | Acceptable range |
|---|---|---|---|---|
| 1 | Tap water | 258.0 g | | |
| 2 | Dipotassium hydrogen phosphate | 17.0 g | 1.70% | 0.70% to 13.90% |
| 3 | Potassium hydroxide | 5.0 g | 0.50% | 0.10% to 5.60% |
| 4 | Chlorous acid aqueous solution (pH 2.9 to 3.5) | 720.0 g | 72.00% | 0.25% to 75% |
| Total | | Chlorous acid 30000 ppm | 1000 g | |

**[Table 5]**

| CAAS Specification | Chlorous acid aqueous solution formulation manufactured with chlorous acid aqueous solution |
|---|---|
| Content | 3.0% |
| Attribute | Yellow |
| Confirmation Test (1) | Match |
| Confirmation Test (2) | Match |
| Confirmation Test (3) | Match |
| Purity Test (1) | Match |
| Purity Test (2) | Match |

For the "chlorous acid aqueous solution formulation manufactured with chlorous acid aqueous solution" prepared based on the preparation method described above, the concentration of "chlorous acid aqueous solution" was measured based on the "Quantification method of chlorous acid aqueous solution" described above, and buffer prepared to achieve the free chlorine concentration described in each Example (phosphate buffer comprising dipotassium hydrogen phosphate or potassium dihydrogen phosphate) was used to prepare the chlorous acid aqueous solutions of each Example.

### (Test for studying the efficacy of chlorous acid aqueous solution)

A Preston medium is prepared.

### Preston medium

(1) 12.5 g of nutrient broth No. 2 medium (composition of 500 mL of medium) is added and gently mixed with 475 mL of distilled water.
(2) The mixture is subjected to autoclave for 15 minutes at 121°C, cooled to about 50°C, and added with polymyxin B sulfate 2500U dissolved in saline.
(3) 5 mg of trimethoprim, 5 mg of rifampicin, and 50 mg of cycloheximide are dissolved in 2 mL of a solvent (acetone: sterilized water = 1:1) and added, thoroughly mixed, and 25 mL of horse hemolysate is added to prepare the medium.
(4) In accordance with the composition described above, 7 L is prepared as a whole while taking into consideration the capacity of an autoclave at the department of fisheries science, the size of an Erlenmeyer flask, etc.
(5) 225 mL of Preston medium is dispensed in 15 + 15 for a total of 30 sterilized containers for 10x Preston medium for the stomach. Subsequently, 75 mL of Preston medium is dispensed in 15 sterilized containers for 4x diluted Preston medium.
(6) 9.9 mL of Preston medium is loaded into 108 plastic tubes for use in a method using three sets and three tubes, 9 mL thereof is loaded into 108 tubes, and control is loaded into 2 tubes.

Since 4.5 L is produced in this test, each of the following agents is used.
Preston medium: 4.5 L
Agents: amount used
Distilled water: 4275 mL
Nutrient broth No. 2 medium: 112.5 g
Polymyxin B sulfate 500000U: 41250U (0.45 mL of solution prepared from dissolving the agent by adding 10 mL of sterilized saline in 1 vial)
Trimethoprim: 45 mg
Rifampicin: 45 mg
Cycloheximide: 450 mg
Organic solvent (acetone:sterilized water = 1:1) *for dissolving antibiotics: 25 mL
Horse hemolysate: 225 mL

### Buffer peptone water

(1) 10.0 g of buffer peptone water (BPW) (composition of 500 mL of medium) is added to and gently mixed with 500 mL of distilled water.
(2) The mixture is subjected to autoclave for 15 minutes at 121°C, cooled to about 50°C, and dispensed in each Erlenmeyer flask.
(3) In accordance with the composition described above, 4 L is prepared as a whole while taking into consideration the capacity of an autoclave at the department of fisheries science, the size of an Erlenmeyer flask, etc.

Since 3.5 L is produced in this test, each of the following agents is used.
Buffer peptone: 3.5 L
Agent: amount used
Distilled water: 3500 mL
Buffer peptide water (BPW): 70 g
   mCCDA medium (SEL formulation): 350
Butzler's medium: 350 of them are prepared.
VRBG medium: 60 of them are purchased.
Nutrient agar medium: 60 of them are purchased.
OF medium: 120 of them are purchased.
Oxidase filter paper: amount required is purchased as needed.

### Preparation 2

Each specimen processed at locations A to D in Figure 1 is withdrawn.
(1) A broiler at first in the morning (operation starts at 7:00) is collected.
(2) Microparticles of a chlorous acid aqueous solution are sprayed from apparatuses at locations A to D.
   The free chlorine concentration of a chlorous acid aqueous solution formulation was checked by using an available chlorine concentration meter RC-V2-HS. By referring to this value, the formulation was diluted to have a free chlorine concentration of 25 mg/L. The diluent was sprayed at a microparticle size of 8 um or less and filled within a certain space through which a chicken is to pass.
(3) A sample was withdrawn at a total of 5 locations, i.e., before and after A, B, C, and D.

N = 3 samples are withdrawn at each location. Since there are samples for Preston medium and samples for buffer peptone water, it is necessary to withdraw 6 carcasses at each location (5 locations × 6 carcasses = total of 30 carcasses)

### (For Preston medium)

(4)-1: The carcass and eviscerated carcass were thoroughly skinned including neck skin, breast skin, thigh skin, and back skin, and the skin was placed in Ziploc^{®} to measure the weight.
(5)-1: A Preston medium (about 60 ml) is placed in the Ziploc^{®} of (4)-1 so that chicken skin:Preston medium = 1:1, and the bag is sealed.
(6)-1: Stomach is processed.
(7)-1: The stomach solution is used as a sample solution for an MPN method.
(8)-1: Chicken skin is retrieved from (7)-1, and the residual solution is recovered.
(9)-1: The entire amount of the residual solution of (8)-1 is transferred to a centrifugation tube and centrifuged. For the conditions of centrifugation, centrifugation is performed for 5 minutes at 4°C after reaching 10000 × g. After the centrifugation, the supernatant (99% or more) is discarded, and the precipitate is recovered.
(10-1): A Preston medium (about 1 ml to 2 ml) at 1/100 of the volume of the residual solution in (9)-1 is placed in the centrifugation tube in (7)-1 and thoroughly dissolved to prepare a sample solution for a quantification method.

### (For buffer peptone water)

(4)-2: The carcass and eviscerated carcass are thoroughly skinned including neck skin, breast skin, thigh skin, and back skin, and the skin is placed in Ziploc^{®} to measure the weight.
(5)-2: Buffer peptone water (about 180 ml) is placed in the Ziploc^{®} of (4)-2 so that chicken skin:buffer peptone water = 1:1, and the bag is sealed.
(6)-2: Stomach is processed.
(7)-2: The stomach solution is used as a sample solution for measuring enterobacteriaceae.

### Test method

### *Measurement of Campylobacter bacteria count through a quantification method

(1) The specimen in (10)-1 is used as an undiluted solution (×1) and serially diluted ×10 and ×100. 0.1 ml of each specimen was smeared onto each sheet of an mCCDA (SEL formulation) medium and Butzler's medium and subjected to microaerobic culture for 48 hours at 42°C.
(2) After the culture, the characteristics of colonies that have grown are observed, and the bacteria count is recorded for each face of a typical colony.
(3) Bacteria are streaked onto an mCCDA medium (SEL formulation) and Butzler's medium and subjected to microaerobic culture for 48 hours at 42°C for each face of a typical colony.
(4) The grown typical colonies are inspected with a phase contrast microscope and subjected to aerobic culture, oxidase test, and catalase test to determine whether bacteria are Campylobacter bacteria. Typical colonies that are highly likely Campylobacter bacteria in view of these results are streaked on an mCCDA medium (SEL formulation) and Butzler's medium for pure culture and subjected to microaerobic culture for 48 hours at 42°C.
(5) Campylobacter bacteria are confirmed by using PCR to find the final actual measured bacteria count in the grown typical colonies.

### *Measurement of Campylobacter bacteria count by a method using three sets and three tubes (MPN method)

(1) The specimen of (7)-1 is used as an undiluted solution (×1). 10 ml thereof is fractionated, and placed in 10 ml × 3 Preston medium. 1 ml of an undiluted solution (×1) is then fractionated and placed in 9 ml × 3 Preston medium. 0.1 ml of an undiluted solution (×1) is then fractionated and placed in 9.9 ml × 3 of Preston medium. These media are subjected to microaerobic culture for 48 hours at 42°C to grow the bacteria.
(2) Bacteria are streaked on an mCCDA medium (SEL formulation) and Butzler's medium from tubes cultured for 48 hours, which were used in a method using three sets and three tubes.

The bacteria are cultured for 48 hours at 42°C. The status of culture in the tubes in a method using three sets and three tubes is recorded.
(3) After culture, the characteristics of colonies that have grown are observed, and the bacteria are streaked onto an mCCDA medium (SEL formulation) and Butzler's medium and subjected to microaerobic culture for 48 hours at 42°C for each face of a typical colony.
(4) The grown typical colonies are inspected with a phase contrast microscope and subjected to aerobic culture, oxidase test, and catalase test to determine whether bacteria are Campylobacter bacteria. Typical colonies that are highly likely Campylobacter bacteria in view of these results are streaked on an mCCDA medium (SEL formulation) and Butzler's medium for pure culture and subjected to microaerobic culture for 48 hours at 42°C.
(5) The grown typical colonies are confirmed to be Campylobacter bacteria using PCR to make the final determination. The MPN value is found by referring to the table for "MPN per 100 ml of sample in three stage dilution of three tubes for each stage and 95% confidence interval thereof".

### *Measurement of the number of enterobacteriaceae

(1) The specimen of (7)-2 is used as an undiluted solution (×0). The specimens withdrawn at locations A, B, and C are serially diluted to undiluted solution (×0), ×2, ×4, and ×6. The specimens withdrawn at location D are serially diluted to undiluted solution (×0), ×1, ×3, and ×5. The specimens withdrawn at locations E and F are serially diluted to undiluted solution (×0), ×1, and ×3.
(2) 0.1 mL of each of the serially diluted solutions is fractionated, seeded in a VRBG medium, and cultured for 24 hours at 37°C.
(3) After the culture, colonies grown in each medium are counted to determine the number of colonies of enterobacteriaceae.
(4) Typical colonies (light red, red, or purple colonies) growing in the VRBG medium are picked and streaked on a nutrient agar medium to check the attribute of individual colonies (oxidase/fermentation glucose test).
(5) After culture, an attribute test is conducted on individual grown colonies.

### Oxidase test

A true platinum line is used to pick an individual colony with an inoculation loop and smeared onto oxidase filter paper.

If filter paper turns dark after about 10 seconds, it is deemed positive, and otherwise negative.

### Fermentation glucose test

An individual colony with a negative oxidase test is picked with an inoculation loop and pierced into two fermentation glucose test media (OF medium). One is directly cultured, and the other is cultured for 24 hours at 37°C after overlaying sterilized liquid paraffin thereabove. If enterobacteriaceae ferments glucose to generate lactic acid, the color tone of the entire medium has turned yellow for both media, and gas (CO₂) is generated from the medium with overlaid liquid paraffin after culture, the fermentation glucose test is positive. However, if both media do not change, the fermentation glucose test is negative. (6) Colonies determined to be negative in the oxidase test and positive in the fermentation glucose test are deemed enterobacteriaceae, and the number of enterobacteriaceae is determined.

### (Example 1-1)

### (Test for studying effectiveness of chemical sterilization treatment on Campylobacter bacteria (C. jejuni/coli) in chicken carcass processed in practice)

Test method: The apparatuses at A, B, and C in Figure 1 (apparatus A, apparatus B, and apparatus C, respectively) were actuated to fill microparticles of a chlorous acid aqueous solution at each location. Broilers at first in the morning that have passed therethrough were withdrawn from a plurality of carcasses or eviscerated carcasses at each point.

Food additive: chlorous acid aqueous solution
Concentration: free chlorine concentration (as chlorine:Cl = 35.45) 25 mg/L (final concentration)
Regulator: 0.4 Mpa
Magnetic drive seal response pump (Iwaki Magnet Pump 15 to 17 L/minute, 100V, 70 to 90 W), limited to those that spray solely through liquid pressure
Period of passing through the space (*not spray period): inside apparatus A: 4 seconds, inside apparatus B: 2 seconds, inside apparatus C: 11 seconds
Line speed: 53.3 fowls/minute
Microparticle size: 8 um or less (size at which hand would not be "wet" upon contact with microparticles)
*On lines where chickens pass through, a certain space on the line is sufficiently filled with microparticles of a chlorous acid aqueous solution that satisfy the microparticle size condition described above, and carcasses or eviscerated carcasses pass therethrough. It should be checked in advance whether a hand is not "wet" upon contact with filled microparticles.

### Test I: Test for studying the effect of reducing Campylobacter bacteria derived from feces (farm)

**[Table 6]**

| | **F farm broiler** | | **M hatchery broiler** | **M farm broiler** |
|---|---|---|---|---|
| **Steps** | **Before operationof apparatus A** | **After operation of apparatus A** | **After operation of apparatus A** | **After operation of apparatus A** |
| **After defeathering** | **460** | **460** | **460** | **140** |
| | **460** | **460** | **240** | **93** |
| **Before apparatus A** | **240** | **240** | **240** | **460** |
| **After apparatus A** | **150** | **29** | **43** | **23** |
| | **240** | **93** | **43** | **93** |
| | **43** | **43** | **43** | **23** |
| **Campylobacter bacteria count (MPN; per 10 g of chicken skin)** | | | | |

The skin on the breast, thigh, and back of collected broilers was completely torn off. 25 g thereof was subjected to stomach processing in a Preston medium, and MPN values (method using three sets and three tubes) were found by a method with the highest sensitivity. Turbidity in a test tube was checked by PCR.

An effect of reducing Campylobacter bacteria derived from feces (farm) was observed from passing a carcass through a space filled with microparticles (mist) of a chlorous acid aqueous solution in apparatus A. A certain effect of reducing Campylobacter bacteria derived from feces (farm) was observed at each of the farms.

### Test II: Test for studying the effect of reducing Campylobacter bacteria derived from organ content (farm)

**[Table 7]**

| | **M hatchery broiler** | **M farm broiler** |
|---|---|---|
| **Steps** | **After operation of each apparatus** | **After operation of each apparatus** |
| **After defeathering** | **460** | **140** |
| **Before apparatus A** | **240** | **93** |
| | **240** | **460** |
| **After apparatus A** | **43** | **23** |
| | **43** | **93** |
| | **43** | **23** |
| **After evisceration** | **240** | **150** |
| | **240** | **240** |
| **Before apparatus B** | | |
| | **460** | **240** |
| **After apparatus C** | **23** | **29** |
| | **93** | **43** |
| | **93** | **23** |
| **Campylobacter bacteria count (MPN; per 10 gof chicken skin)** | | |

The Campylobacter bacteria count that was first reduced by apparatus A increases again after going through an evisceration step. The bacteria count was able to be reverted back to the bacteria count before evisceration after passing through apparatus B→inside/outside cleaning→apparatus C. However, the bacteria count only reverts back to the number of Campylobacter bacteria derived from feces (farm) reduced by apparatus A.

Campylobacter bacteria derived from organ content (farm) that has adhered during an evisceration step are readily removed because the bacteria are merely adhering to the outside. It is important how much Campylobacter bacteria derived from feces (farm) can be reduced.

### Test III: Effect of reducing Campylobacter bacteria after a cooling tank

**[Table 8]**

| | **M hatchery broiler** | **M farm broiler** |
|---|---|---|
| **Steps** | **After operation of each apparatus** | **After operation of each apparatus** |
| **After defeathering** **Before apparatus A** | **460** | **140** |
| | **240** | **93** |
| | **240** | **460** |
| **After apparatus A** | **43** | **23** |
| | **43** | **93** |
| | **43** | **23** |
| **After evisceration** **Before apparatus B** | **240** | **150** |
| | **240** | **240** |
| | **460** | **240** |
| **After apparatus C** | **23** | **29** |
| | **93** | **43** |
| | **93** | **23** |
| **After cooling** **Before apparatus D** | **<3.6** | **<3** |
| **Campylobacter bacteria count (MPN; per 10 g of chicken skin)** | | |

Campylobacter bacteria was hardly detected from an eviscerated carcass after cooling. If Campylobacter bacteria can be reduced with a cooling tank, it may be possible that conventional processing methods are sufficient without using a chlorous acid aqueous solution. Thus, a sterilization effect of sodium hypochlorite, which is automatically titrated in cooling water, was checked. The values before apparatus D after cooling all correspond to "at or below detection limit" of positive tube count (0, 0, 0) .

### (Example 1-2)

### (Test of studying efficacy of chemical sterilization treatment on Campylobacter bacteria (C. jejuni/coli) throughout the year)

Test method: The same test as Example 1-1 was conducted in
the fall: October 26, 2018, winter: February 10, 2019, and
summer: July 26, 2019. The same test was conducted three times on each test day.
Food additive: chlorous acid aqueous solution
Concentration: free chlorine concentration (as chlorine:Cl = 35.45) 25 mg/L (final concentration)
Regulator: 0.4 Mpa
Magnetic drive seal response pump (Iwaki Magnet Pump 15 to 17 L/minute, 100V, 70 to 90 W), limited to those that spray solely through liquid pressure
Period of passing through the space (*not spray period): inside apparatus A: 4 seconds, inside apparatus B: 2 seconds, inside apparatus C: 11 seconds
Line speed: 53.3 fowls/minute
Microparticle size: 8 um or less (size at which hand would not be "wet" upon contact with microparticles)
*On lines where chickens pass through, a certain space on the line is sufficiently filled with microparticles of a chlorous acid aqueous solution that satisfy the microparticle size condition described above, and carcasses or eviscerated carcasses pass therethrough. It should be checked in advance whether a hand is not "wet" upon contact with filled microparticles.

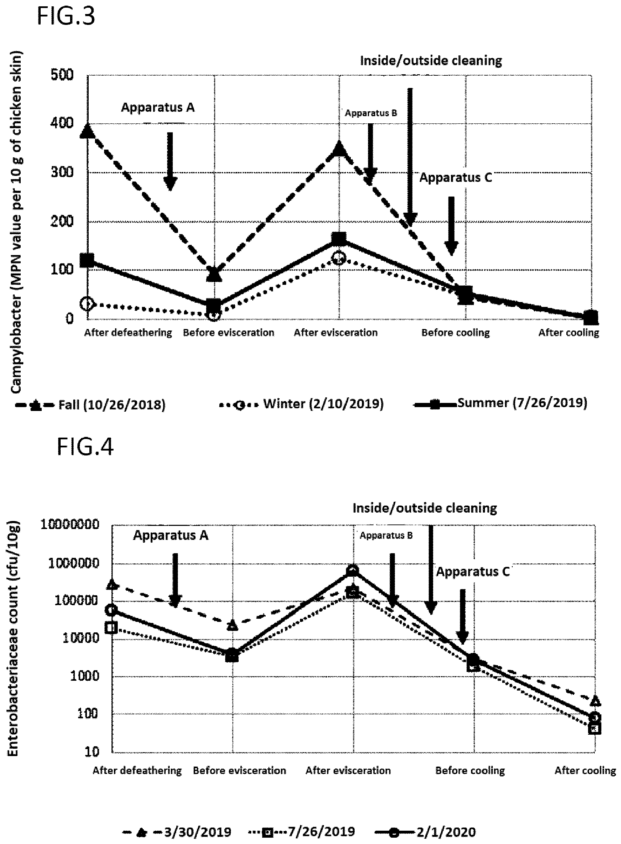

As the obtained values, the mean value of three tests conducted on each test day was used and summarized as a graph in Figure **3****.**

The Campylobacter bacteria count dramatically decreased through a sterilization processing step by apparatus A from after defeathering to before evisceration, and the bacteria count increased again after the subsequent evisceration. The bacteria count then decreased by apparatus B and apparatus C before cooling. After cooling following passage through a chiller tank, the bacteria count was able to be reduced to or below the detection limit value (positive tube count 0,0,0). The same effect is always observed throughout the year. In particular, it was unprecedented that the number of Campylobacter bacteria adhering to chicken skin in the summer, i.e., July 26, was able to be reduced to or below the detection limit.

### (Example 1-3)

### (Test of studying efficacy of chemical sterilization treatment on enterobacteriaceae)

Test method: A test was conducted three times on enterobacteriaceae under the same condition as Example 1-1.
Food additive: chlorous acid aqueous solution
Concentration: free chlorine concentration (as chlorine:Cl = 35.45) 25 mg/L (final concentration)
Regulator: 0.4 Mpa
Magnetic drive seal response pump (Iwaki Magnet Pump 15 to 17 L/minute, 100V, 70 to 90 W), limited to those that spray solely through liquid pressure
Period of passing through the space (*not spray period): inside apparatus A: 4 seconds, inside apparatus B: 2 seconds, inside apparatus C: 11 seconds
Line speed: 53.3 fowls/minute
Microparticle size: 8 um or less (size at which hand would not be "wet" upon contact with microparticles)
*On lines where chickens pass through, a certain space on the line is sufficiently filled with microparticles of a chlorous acid aqueous solution that satisfy the microparticle size condition described above, and carcasses or eviscerated carcasses pass therethrough. It should be checked in advance whether a hand is not "wet" upon contact with filled microparticles.

As the obtained values, the mean value of three tests conducted on each test day was used and summarized as a graph in Figure **4****.**

Just like Campylobacter bacteria, enterobacteriaceae count decreased through a sterilization processing step by apparatus A from after defeathering to before evisceration, and the bacteria count increased again after the subsequent evisceration step. The bacteria count then decreased by apparatus B and apparatus C before cooling. After cooling following passage through a chiller tank, the bacteria count was able to be reduced to the order of 10². The same effect is always observed throughout the year.

However, it appears that Campylobacter bacteria and enterobacteriaceae can be reduced only after going through the cooling step, instead of a sterilization step of spraying microparticles using a chlorous acid aqueous solution. Meanwhile, sodium hypochlorite that is automatically titrated in chiller water is for preventing contamination of chiller water and cannot be used for sterilization. This is because microorganisms that are present in an eviscerated carcass are inside of the eviscerated carcass surface. It is known that an increase in the amount of sodium hypochlorite in chiller water does not affect the microorganisms within the top surface of an eviscerated carcass or lead to a reduction effect. Examples 2 and 3 were conducted for the verification thereof.

### (Example 2)

### (Test for studying the relationship of available chlorine concentration, free chlorine concentration, and turbidity of sodium hypochlorite that is automatically titrated in cooling water)

Agents used
Sodium hypochlorite, available chlorine *1): 12% product, 20 L (commercially available product)
Conditions of each chiller tank
*Volume of each chiller tank
Volume of pre-cooling chiller tank: 6000 L (6 t)
Volume of main cooling chiller tank: 22200 L (22.2 t)
*Cooling water is always filled in the amount of the volume

### Flow of cooling water

Cooling water enters a main cooling chiller tank at 5 t per hour (5 t/h) from the main cooling chiller tank side. The 5 t/h of cooling water then flows into a pre-cooling chiller tank from the main cooling chiller tank. Lastly, the 5 t/h of cooling water overflows and is discharged outside from the pre-cooling chiller tank.

### Automatic titration of sodium hypochlorite

As sodium hypochlorite, a 12% product (about 100,000 ppm to 120,000 ppm), is used. An undiluted solution of the sodium hypochlorite is directly added, once at 9:00 AM and once before afternoon for a total of two times from the location of a star symbol to the main cooling chiller tank side at a volume of 15 L and to the pre-cooling chiller tank side at a volume of 10 L. Further, an undiluted solution of the sodium hypochlorite is always stored within a titrator tank, and the solution is automatically titrated when appropriate. An undiluted solution of sodium hypochlorite consumed in one day is determined to be 90 L/day in the main cooling chiller tank and 65 L/day in the pre-cooling chiller tank. The available chlorine of sodium hypochlorite is always adjusted with an automatic titrator to be 100 ppm or greater.

The cooling step after the main sterilization is separated into a pre-cooling chiller tank (6 t) and a main cooling chiller tank (22.2 t). Cooling water of 5 t per hour flows into the pre-cooling chiller tank from the main cooling chiller tank and ultimately overflows and is discharged. A certain amount of 12% (100,000 to 120,000 ppm) sodium hypochlorite is added to the cooling water into the main cooling chiller tank and pre-cooling chiller tank twice, i.e., at 9:00 AM and before the start of the afternoon shift. The concentration thereafter is always adjusted to maintain 100 ppm or greater as the available chlorine concentration by automatic titration when appropriate by using an automatic titrator, but the free (residual) chlorine concentration (value obtained by the DPD method), which has a cause and effect relationship with the sterilization effect, is not managed.

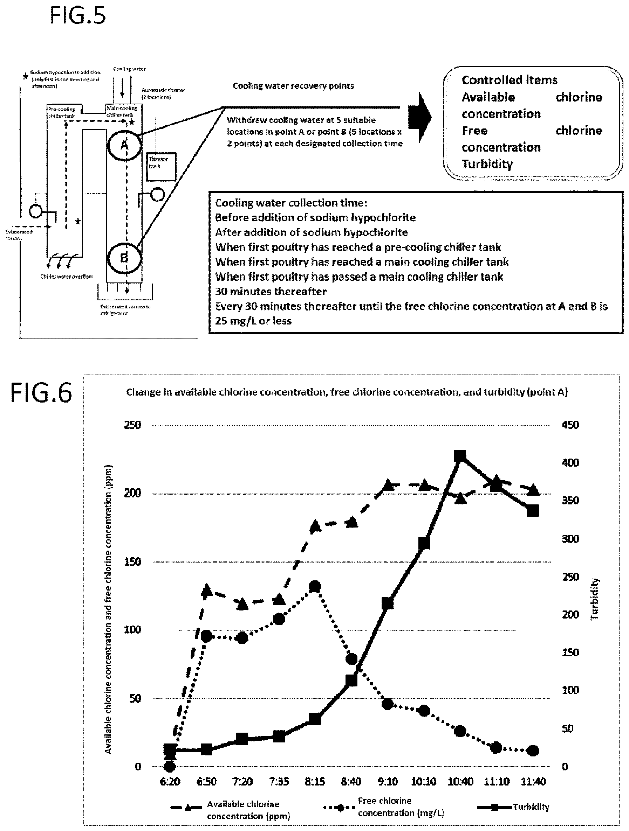

In this regard, sodium hypochlorite was added in advance to each chiller water. The available chlorine and free chlorine concentrations and the change thereof overtime were measured by using a residual chlorine meter RC-V2 (Kasahara Chemical Instruments Corp.). A schematic diagram of a cooling water collection method is shown in Figure **5**.

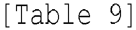

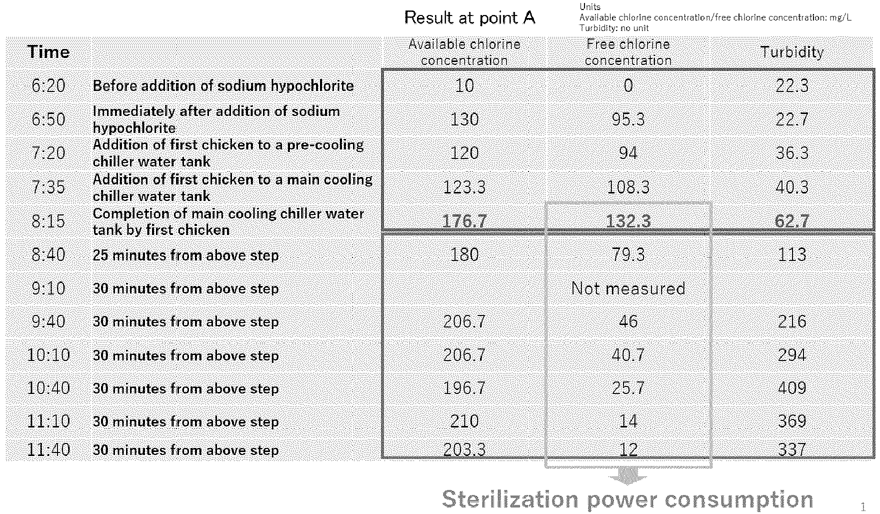

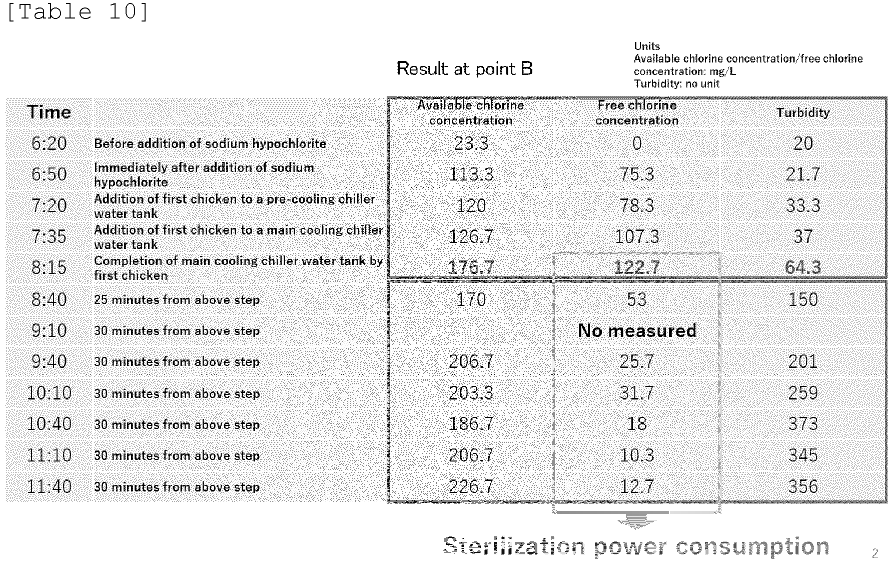

The relationship of the free chlorine concentration, available chlorine concentration, and turbidity at point A is summarized in a graph (Figure **6**).

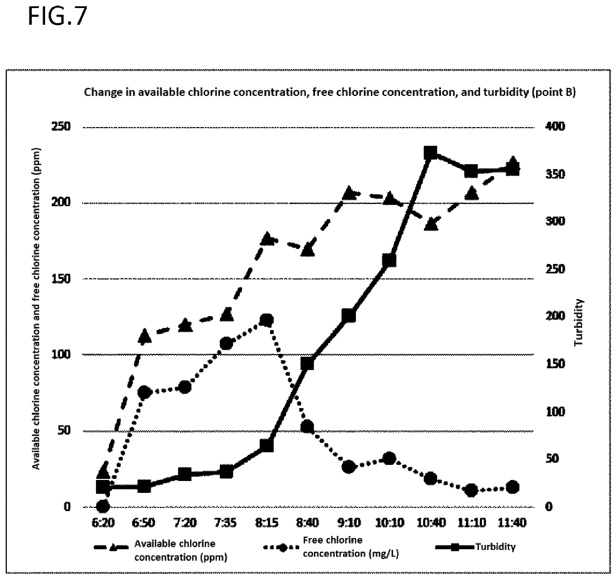

The relationship of the free chlorine concentration, available chlorine concentration, and turbidity at point B is summarized in a graph (Figure **7**).

Since sodium hypochlorite is continuously added by an automatic titrator, the available chlorine concentration continued to increase with passage of time, but turbidity was observed to increase once an eviscerated carcass was added. However, a correlation between turbidity and available chlorine concentration of sodium hypochlorite was not found.

Meanwhile, a phenomenon where the free chlorine concentration rapidly decreases immediately after turbidity increases to a certain level was observed. It was found that a free chlorine concentration is a value that can be an indicator of sterilization power, and sterilization power decreases significantly as turbidity increases (inverse correlation).

It was also found that the free chlorine concentration was mostly eliminated three hours after the eviscerated carcass has been moved out from the main cooling chiller tank. It was demonstrated that a sterilization effect of sodium hypochlorite was not exerted, despite the continuous addition of sodium hypochlorite to the chiller tank. In view of the above result, sterilization power is completely lost only after three hours from moving the eviscerated carcass out from the chiller tank, despite the available chlorine concentration being maintained at a high concentration.

Moreover, sodium hypochlorite in a chiller tank is controlled by available chlorine concentration in quality control items. While it appears that the available chlorine concentration is maintained at 200 ppm or greater in quality control, the results show that the sterilization power in cooling water is in fact completely lost, and sterilization effect cannot be expected from sodium hypochlorite that is automatically titrated in chiller water. In addition, it is necessary to quickly discontinue a generally accepted management system that focuses only on the change in available chlorine concentration and to change the system to a system for managing the change in free chlorine concentration.

### (Example 3)

### (Test for studying the sterilization effect of a chlorous acid aqueous solution and sodium hypochlorite added to pseudo-cooling water)

Objective: an eviscerated carcass immediately after an evisceration step is inoculated with enterobacteriaceae to investigate the extent of decrease in a sterilization effect on enterobacteriaceae after immersion in pseudo-cooling water including a chlorous acid aqueous solution formulation or sodium hypochlorite diluted to a suitable free chlorine concentration for 50 minutes.

Eviscerated carcasses of 14 poultry were collected.

Two poultry were used in a blank segment, two poultry were used in a control segment, and the remaining 10 poultry were used in a test segment.

The top skin was completely torn off in the blank segment at 150 g each and was added so that top skin (150 g): buffer peptone (150 mL) = 1:1, and the stomach was treated for 1 minute. After appropriate serial dilution, 0.1 mL each was smeared on a VRBG medium and cultured for 24 hours at 37°C.

For the control segment, a mixed bacterial solution of enterobacteriaceae (E. coli, Enterobacter, Citrobacter) was prepared and sprayed on eviscerated carcasses of two poultry in the control segment, which were left standing for 5 minutes. The top skin was completely torn off at 150 g each and was added so that top skin (150 g): buffer peptone (150 mL) = 1:1, and the stomach was treated for 1 minute. After appropriate serial dilution, 0.1 mL each was smeared on a VRBG medium and cultured for 24 hours at 37°C.

For the experiment segment, a mixed bacterial solution of enterobacteriaceae (E. coli, Enterobacter, Citrobacter) was sprayed, and the poultry was left standing for 5 minutes. The poultry was then immersed for 50 minutes in a barrel containing pseudo-cooling water (4°C or less, solid-liquid ratio = 1:10) including a chlorous acid aqueous solution or sodium hypochlorite with a free chlorine concentration of 5 mg/L, 25 mg/L, 50 mg/L, or 100 mg/L. The top skin was completely torn off from the recovered eviscerated carcasses at 150 g each and was added so that top skin (150 g): buffer peptone (150 mL) = 1:1, and the stomach was treated for 1 minute. After appropriate serial dilution, 0.1 mL each was smeared on a VRBG medium and cultured for 24 hours at 37°C.

The results are shown below.

**[Table 11]**

| **Specimen name** | | | **Enterobacteriaceae** | | **Free chlorine concentration** | |
|---|---|---|---|---|---|---|
| | | | **Bacterial count (cfu/g)** | **Percentage of reduction (cfu/g)** | **Residual chlorine (mg/L)** | **Residual percentage (%)** |
| **Inoculated bacterial solution** | | | **1.3 × 10^8** | **-** | **Not measured** | **-** |
| **Blank segment** | **Bacterial solution not inoculated** | | **4.4 × 10^6** | **-** | **Not measured** | **-** |
| **Control segment** | **Bacterial solution (no sterilization)** | | **1.8 × 10^7** | **-** | **Not measured** | **-** |
| **Test segment** | **Sodium hypochlorite** | **5mg/L** | **1.9 × 10^7** | **0** | **0.0** | **0.0** |
| | | **25mg/L** | **1.5 × 10^7** | **0** | **1.0** | **4.0** |
| | | **50mg/L** | **1.8 × 10^6** | **10^1** | **11.0** | **22.0** |
| | | **100mg/L** | **1.3 × 10^6** | **10^1** | **49.5** | **49.5** |
| | **Chlorous acid aqueous solution** | **5mg/L** | **7.4 × 10^6** | **10^1** | **4.0** | **80.0** |
| | | **25mg/L** | **6.2 × 10^4** | **10^3** | **16.0** | **64.0** |
| | | **50mg/L** | **1.6 × 10^4** | **10^3** | **40.0** | **80.0** |
| | | **100mg/L** | **2.7 × 10^4** | **10^3** | **80.0** | **80.0** |

The free chlorine concentration of sodium hypochlorite was significantly lost. A sterilization effect was not observed at all at a free chlorine concentration of 25 mg/L or less. A percentage of reduction of about 10¹ cfu/g was observed at a free chlorine concentration of 50 mg/L or greater. It was found therefrom that cooling water of a chiller tank needs to continuously maintain at least 50 mg/L of free chlorine concentration. However, the sterilization effect of 10¹ cfu/g indicates that bacteria count has reverted back to that before inoculation of bacterial solution in the eviscerated carcass. It was found that even if bacteria adhering to the surface of the eviscerated carcass could be removed with cooling water, cooling water has no effect on enterobacteriaceae that are latent in the inside of the carcass.

Meanwhile, a chlorous acid aqueous solution was found to have a percentage of reduction of 10³ cfu/g by maintaining a free chlorine concentration at 25 mg/L or greater. This indicates that a chlorous acid aqueous solution can sterilize not only enterobacteriaceae that are adhering to the surface of the eviscerated carcass, but also enterobacteriaceae that are latent inside the eviscerated carcass. One of the reasons thereof is in materializing high residual percentage of free chlorine concentration relative to sodium hypochlorite even after contact with an organic matter.

In view of the above, by the time an eviscerated carcass that was placed first is taken out from a chiller tank, cooling water in the chiller tank, which should be automatically titrated, has a free chlorine concentration reduced to 50 mg/L or less, so that even enterobacteriaceae that has adhered during an evisceration step cannot be sterilized. This proves that it is necessary to spray microparticles of a chlorous acid aqueous solution and reduce pathogenic microorganisms in the top surface of a carcass as much as possible while the carcass is warm and then pass the surface of the eviscerated carcass contaminated due to destruction of an organ in the evisceration step through a chiller tank after attaching microparticles of a chlorous acid aqueous solution to the surface of the eviscerated carcass with apparatus B and apparatus C in order to reduce the pathogenic microorganisms adhering to the eviscerated carcass after a chiller step to or below the detection limit, and sodium hypochlorite that is automatically titrated in chiller water cannot reduce pathogenic microorganisms that are latent in the eviscerated carcass.

In view of these Examples, conceivable causes of contamination in each step and measures against them are summarized.

**[Table 12]**

| Number | Location of microorganisms contaminations | Conceivable cause of contamination | Measures |
|---|---|---|---|
| ① | Chicken farm | *Environment of facility (cleanliness, stress due to small cage, etc.) | *Cleaning or disinfection of facility, and adjustment of gut flora of chicken (probiotics or prebiotics, use of antibiotics) |
| | | *Feces from insects, animals, birds, etc., Infections | |
| | | *Contamination of feed or water | *Removal of insects, animals, birds, etc. |
| | | | *Testing and Improving feed and water |
| ② | Transport/loading | *Contamination of chicken in the bottom case due to feces of chicken in the top cage of stacked cages | *Starving of chicken prior to transport from a chicken farm to reduce the amount of feces in the body (12 to 24 hours) |
| | | *Rinsing with water from top of the cage during summer | *Thorough cleaning to remove feces |
| | | *Stress due to small cage (would be a cause of disease) | *Cleaning/disinfection of facility |
| | | *Contamination of facility such as receiving dock, and secondary contamination of chicken | |
| ③ | Broiler | *Cross-contamination due to contact of contaminated chicken with one another | *Re-cleaning of chicken that is not sufficiently cleaned or adding the chicken to the line last |
| | | *Contamination to human when hanging chicken from a shackle, and secondary contamination of chicken from a human | *Cleaning or disinfection of shackles (during operation of steps) |
| ④ | Slaughter, blood letting | *Cross-contamination of chicken due to blood | *Cleaning chicken after slaughter or blood letting |
| | | *Contamination of human, and secondary contamination of chicken from a human | *Use of protective gear by operators |
| | | | *Cleaning/disinfection of facility or equipment (conveyor belt, etc.): (during operation of steps) |
| | | *Contamination of facility or equipment, and secondary contamination of chicken from a human | |
| ⑤ | Scalding | *(2) Hot water contamination due to chicken in the bottom cage contaminated upon transport entering a scalding tank | *Record temperature of hot water (temperature (63 to 64°C) and time thereof) |
| | | | *Exchange contaminated hot water (it is desirable that the amount of water exchanged is 1 L/chicken) |
| | | *Cross-contamination of chicken due to contaminated hot water | |
| | | *No sterilization due to decrease in hot water temperature | *Cleaning/disinfection of scalding tank |
| | | *Contamination of facility, and secondary contamination of chicken or human | *Adjustment of temperature (check microorganism sterilization temperature and time (D value)) |
| ⑥ | Defeathering | *Cross-contamination due to contact of chicken with one another | *Sterilization or disinfection after defeathering (pre-processing cleaning: apparatus X) |
| | | *Contamination of equipment, and secondary contamination of chicken or human | |
| | | | *Cleaning/disinfection of defeathering machine |
| ⑦ | Head/rear limb severance | *Cross-contamination of chicken due to blood or severed parts, and contamination of human | *Use of protective gear by operators |
| | | | *Cleaning/disinfection of equipment |
| | | *Contamination of equipment, and secondary contamination of chicken or human | |

| Number | Location of microorganism | Conceivable cause of contamination | Measures |
|---|---|---|---|
| ⑧ | Evisceration | *Contamination due to damage to organs (intestinal tract) due to eviscerator | *Removal of organ content with jet spray cleaner using water pressure during inside/outside cleaning |
| | | | *Use of a chlorous acid aqueous solution formulation for inside/outside cleaning |
| | | *Cross-contamination and secondary contamination of chicken or human due to contamination of eviscerator | *Improvement of apparatus Z in the main sterilization |
| | | | *Cleaning/disinfection of eviscerator |
| | | | *Cleaning/disinfection of equipment used |
| ⑨ | inside/outside cleaning | *Contamination remaining due to insufficient cleaning | *Removal of organ content with jet spray cleaner using water pressure during inside/outside cleaning |
| | | | *Use of a chlorous acid aqueous solution formulation for inside/outside cleaning |
| ⑩ | Chiller | *Decrease in free chlorine concentration (increase In turbidity) and Increase in pH due to hard substance contained in cooling water or organic matter derived from eviscerated carcass | *Add additional sodium hypochlorite or chlorous acid aqueous solution formulation (need verification test) |
| | | | *Inspect pH, ORP, etc., or check water quality |
| ⑪ | | *Secondary contamination due to contamination of refrigerator | *Cleaning or disinfection of refrigerator |
| | Refrigeration of carcass | | *Absorption of drip with activated carbon, ceramics, etc. |
| | | *Contamination due to drip during refrigeration | *Wrapping carcass with absorbent fabric, or absorbent polymer such as diapers, to absorb drip |
| ⑫ | Disassembly/processing | *Contamination or secondary contamination due to Instrument, equipment, etc. | *Cleaning or disinfection of refrigerator |
| ⑬ | Shipping | *Contamination due to drip during transport | *Absorption of drip with activated carbon, ceramics, etc. |
| | | | *Wrapping carcass with absorbent fabric, or absorbent polymer such as diapers, to absorb drip |
| | | | *Proton freezing (rapidly freezing a subject using even flux or electromagnetic wave and create a large number of fine Ice crystals Instead of a large Ice crystal upon freezing to prevent disruption of cells in food and reduce drip upon thawing) |
| ⑭ | Retail store | *Secondary contamination due to contamination of refrigerator | *Cleaning or disinfection of refrigerator |
| | | | *Absorption of drip with activated carbon, ceramics, etc. |
| | | | *Wrapping carcass with absorbent fabric, or absorbent polymer such as diapers, to absorb drip |
| | | *Contamination due to drip during refrigeration | *Exchanging wrapping or container used in storage |
| | | | *Determining an expiration date and refraining from providing products that are not fresh to customers |

Raw materials of chicken dishes that are raw or nearly raw which can pass microorganism control criteria proposed below can be produced by determining critical control points based on analysis of these risk factors and introducing them into HACCP.

"At or below detection limit", bacteria count can be deemed to be at a level where food poisoning incidents due to enterobacteriaceae including Campylobacter bacteria would not occur. "At or below detection limit" is a state where Campylobacter bacteria would not grow at all even if grown in a culture, i.e., state of positive tube count (0, 0, 0) .

It is demonstrated that the bacteria count can be reduced to much less than 300 per 1 g of chicken skin of eviscerated carcass, which is the standard value that is generally considered "negative", or less throughout the year in accordance with the claims herein.

Specifically, raw materials of chicken meat that can be used for poultry meat for raw consumption can be provided by incorporating the concepts of specification and standards for poultry meat for raw consumption (microorganism control standards) and schematics of introducing a chlorous acid aqueous solution formulation mist spraying sterilization apparatus based on HACCP for the materialization thereof that are proposed herein.

Concepts of specification and standards for poultry meat for raw consumption (microorganism control standards) and schematics of introducing a chlorous acid aqueous solution formulation mist spraying sterilization apparatus based on HACCP for the materialization thereof are the items recited in the claims.

Specification and standards for poultry meat for raw consumption (microorganism control standards) are the following.

### (Microorganism control standards)

*Number of enterobacteriaceae: negative (in 25 g of final product) (based on testing method of the ISO methodology (21528)
*Campylobacter bacteria:
   negative (according to quantification method (mCCDA (SEL) medium method))
   50 MPN (in 100 g of final product) (according to a method using three sets and three tubes)
      *Salmonella bacteria: 0.014 cfu/g or less (based on specification and standards for meat (beef) for raw consumption)
      *Enterohemorrhagic E. coli: 0.014 cfu/g or less (based on specification and standards for meat (beef) for raw consumption)

However, the distribution temperature is limited to 5°C or lower for raw materials of chicken meat satisfying microorganism control standards.

As described above, the present invention is exemplified by the use of its preferred embodiments. It is understood that the scope of the present invention should be interpreted solely based on the claims. The present application claims priority to Japanese Patent Application No. 2020-120838 (filed on July 14, 2020). The entire content thereof is incorporated herein by reference. It is understood that any patent, any patent application, and any references cited herein should be incorporated herein by reference in the same manner as the contents are specifically described herein.

### [Industrial Applicability]

The method of the present disclosure provides a method of manufacturing a chicken meat raw material that can also be used in chicken meat products for raw consumption.

## Claims

1. A method of manufacturing poultry meat, comprising a step of contacting a chlorous acid aqueous solution with a meat raw material of a fowl for consumption.

2. The method of claim 1, comprising a step of contacting a chlorous acid aqueous solution with the fowl after removing an organ from the fowl.

3. The method of claim 1 or 2, further comprising a step of contacting a chlorous acid aqueous solution after defeathering the fowl and before removing an organ from the fowl.

4. The method of any one of claims 1 to 3, further comprising a step of contacting a chlorous acid aqueous solution after removing an organ from the fowl, contacting a chlorous acid aqueous solution with the fowl, and cleaning inside and outside of the fowl with a chlorous acid aqueous solution supplemented solution and before cooling the fowl.

5. The method of any one of claims 1 to 4, further comprising a step of contacting a chlorous acid aqueous solution after cooling the fowl and before refrigeration.

6. The method of any one of claims 1 to 5, comprising a step of cleaning inside and outside of the fowl, wherein the step of cleaning inside and outside of the fowl comprises a step of sterilizing a microorganism derived from an organ while rinsing off organ content adhering to a surface with a chlorous acid aqueous solution having a free chlorine concentration of 1 to 200 mg/L.

7. The method of any one of claims 1 to 6, wherein a microorganism adhering to a surface of a fowl is sterilized with a chlorous acid aqueous solution while cooling.

8. The method of claim 7, wherein a free chlorine concentration of a chlorous acid aqueous solution in the cooling is 1 to 200 mg/L.

9. The method of any one of claims 1 to 8, wherein the step of contacting a chlorous acid aqueous solution also comprises a step of contacting a microparticulate chlorous acid aqueous solution.

10. The method of claim 9, wherein the step of contacting a chlorous acid aqueous solution comprises a step of passing the fowl through a space where a microparticulate chlorous acid aqueous solution is accumulated.

11. The method of claim 9 or 10, wherein a mean particle size of the microparticulate chlorous acid aqueous solution is 1 um to 10 um.

12. The method of any one of claims 1 to 11, wherein a free chlorine concentration of the chlorous acid aqueous solution is 1 mg/L to 200 mg/L.

13. The method of any one of claims 1 to 12, wherein the poultry meat is poultry meat that can be used not only as a conventional chicken meat raw material, but also as a raw material of a chicken meat product for raw consumption.

14. The method of any one of claims 1 to 13, wherein the fowl is a parent stock (spent hen), broiler, roaster, or jidori (free range chicken).

15. The method of any one of claims 1 to 14, wherein the method results in enterobacteriaceae adhering to the poultry meat which is negative in 25 g of a final product, Campylobacter bacteria which are negative according to a quantification method (mCCDA (SEL) medium method) and 50 MPN or less in 100 g of a final product according to a method using three sets and three tubes, Salmonella bacteria which are 0.014 cfu/g or less, and enterohemorrhagic E. coli which is 0.014 cfu/g or less.

16. The method of claim 15, wherein the enterobacteriaceae is a group of bacteria revealed based on a testing method according to the ISO methodology (21528).

17. The method of claim 16, wherein the Campylobacter bacteria are C. coli, C. concisus, C. curvus, C. fetus, C. gracilis, C. helveticus, C. hominis, C. hyointestinalis, C. insulaenigrae, C. jejuni, C. lanienae, C. lari, C. mucosalis, C. rectus, C. showae, C. sputorum, or C. upsaliensis.

18. The method of claim 16, wherein the Salmonella bacteria are S. abortusequi, S. abortusovis, S. typhisuis, S. pullorum, S. gallinarum, S. abortusbovis, S. typhi, S. paratyphi A, S. paratyphi B, S. Bongori, S. typhimurium, S. enteritidis, S. choleraesuis, S. sendai, S. oranienburg, S. chester, or S. arizonae.

19. The method of claim 16, wherein the enterohemorrhagic E. coli is E. coli 0157, E. coli O26, or E. coli O111.

20. The method of any one of claims 1 to 19, wherein a step of contacting a chlorous acid aqueous solution is performed in every step of the steps.

21. A method of producing poultry meat, comprising:
a step of providing meat of a fowl for consumption; and
a step of contacting the meat of a fowl with a chlorous acid aqueous solution to produce poultry meat.

22. The method of claim 21, wherein the method results in enterobacteriaceae adhering to the poultry meat which is negative in 25 g of a final product, Campylobacter bacteria which are negative according to a quantification method (mCCDA (SEL) medium method) and 50 MPN or less in 100 g of a final product according to a method using three sets and three tubes, Salmonella bacteria which are 0.014 cfu/g or less, and enterohemorrhagic E. coli which is 0.014 cfu/g or less.

23. The method of claim 22, wherein the enterobacteriaceae is a group of bacteria revealed based on a testing method according to the ISO methodology (21528).

24. The method of claim 22, wherein the Campylobacter bacteria are C. coli, C. concisus, C. curvus, C. fetus, C. gracilis, C. helveticus, C. hominis, C. hyointestinalis, C. insulaenigrae, C. jejuni, C. lanienae, C. lari, C. mucosalis, C. rectus, C. showae, C. sputorum, or C. upsaliensis.

25. The method of claim 22, wherein the Salmonella bacteria are S. abortusequi, S. abortusovis, S. typhisuis, S. pullorum, S. gallinarum, S. abortusbovis, S. typhi, S. paratyphi A, S. paratyphi B, S. Bongori, S. typhimurium, S. enteritidis, S. choleraesuis, S. sendai, S. oranienburg, S. chester, or S. arizonae.

26. The method of claim 22, wherein the enterohemorrhagic E. coli is E. coli 0157, E. coli O26, or E. coli O111.

27. The method of any one of claims 21 to 26, wherein the poultry meat is poultry meat that can be used not only as a conventional chicken meat raw material, but also as a raw material of a chicken meat product for raw consumption.

28. A method of providing a poultry meat dish comprising a step of preparing a poultry meat dish using poultry meat produced by the method of any one of claims 21 to 27.

29. A chlorous acid aqueous solution having a mean particle size of 1 um to 10 um.

30. A sterilizing agent comprising a microparticulate chlorous acid aqueous solution.

31. The sterilizing agent of claim 30, wherein the sterilizing agent is for sterilizing Campylobacter bacteria and enterobacteriaceae.

32. The sterilizing agent of claim 31, wherein the Campylobacter bacteria are C. coli, C. concisus, C. curvus, C. fetus, C. gracilis, C. helveticus, C. hominis, C. hyointestinalis, C. insulaenigrae, C. jejuni, C. lanienae, C. lari, C. mucosalis, C. rectus, C. showae, C. sputorum, or C. upsaliensis.

33. The method of claim 31, wherein the enterobacteriaceae is a group of bacteria revealed based on a testing method according to the ISO methodology (21528).

34. A method of manufacturing a microparticulate chlorous acid aqueous solution by supplying a chlorous acid aqueous solution at an amount of 0.1 L/hour to 10.0 L/hour to a nozzle having a diameter of 5 um to 15 um, at an amount of air of 10 L/minute to 60 L/minute and at a pressure of 0.04 MPa to 1.2 MPa.

35. The method of claim 34, wherein an available concentration of a chlorous acid aqueous solution is 1 mg/L to 50 mg/L.

36. The method of claim 34 or 35, wherein a spraying time is 2 seconds to 30 seconds.

37. The method of any one of claims 34 to 36, wherein a mean particle size of a chlorous acid aqueous solution is 8 um or less.

38. The method of any one of claims 34 to 37, wherein a shape of a microparticle of the chlorous acid aqueous solution is round, oval, diamond, or omnidirectional.

39. The method of any one of claims 34 to 38, wherein a spraying speed is 0.39 L/minute to 3.2 L/minute.

40. The method of any one of claims 34 to 39, wherein a spraying speed is 0.9 L/hour to 2.0 L/hour.

41. An apparatus for manufacturing a microparticulate chlorous acid aqueous solution, comprising a nozzle having a diameter of 1 um to 10 um and means for providing a chlorous acid aqueous solution at an amount of 0.1 L/hour to 10.0 L/hour, at an amount of air of 10 L/minute to 60 L/minute and at a pressure of 0.04 MPa to 1.2 MPa.

42. The apparatus of claim 41, wherein an available concentration of a chlorous acid aqueous solution is 1 mg/L to 50 mg/L.

43. The apparatus of claim 41 or 42, wherein a spraying time is 2 seconds to 30 seconds.

44. The apparatus of any one of claims 41 to 43, wherein a mean particle size of a chlorous acid aqueous solution is 8 um or less.

45. The apparatus of any one of claims 41 to 44, wherein a shape of a microparticle of the chlorous acid aqueous solution is round, oval, diamond, or omnidirectional.

46. The apparatus of any one of claims 41 to 45, wherein a spraying speed is 0.39 L/minute to 3.2 L/minute.

47. The apparatus of any one of claims 41 to 46, wherein a spraying speed is 0.9 L/hour to 2.0 L/hour.

48. A system for sterilizing meat of a fowl for consumption, comprising:
a spray nozzle for spraying a chlorous acid aqueous solution;
an instrument for providing the meat of a fowl;
means for moving the instrument; and
a barrier for forming a processing space for blocking a side surface with respect to a direction of movement of the instrument.

49. The system of claim 48, further comprising a spraying zone for spraying a chlorous acid aqueous solution.

50. The system of claim 48 or 49, wherein the spray nozzle provides a chlorous acid aqueous solution having a mean particle size of 1 um to 10 um.

51. The system of any one of claims 48 to 50, wherein the sterilization of the meat of a fowl is sterilization of poultry meat that can be used not only as a conventional chicken meat raw material, but also as a raw material of a chicken meat product for raw consumption.

52. A system for manufacturing poultry meat, comprising:
at least one unit selected from the group consisting of a blood-letting unit, a scalding unit, a defeathering unit, an evisceration unit, an inside/outside cleaning unit, a cooling unit, and a refrigeration unit; and
a unit or means for contacting a chlorous acid aqueous solution.

53. The system of claim 52, wherein the unit for contacting a chlorous acid aqueous solution comprises a unit for spraying a chlorous acid aqueous solution.

54. The system of claim 52 or 53, wherein the unit for contacting a chlorous acid aqueous solution comprises a unit comprising a chlorous acid aqueous solution supplemented solution.

55. The system of any one of claims 52 to 54, wherein cleaning water used in the inside/outside cleaning unit comprises a chlorous acid aqueous solution.

56. The system of any one of claims 52 to 55, wherein cooling water used in the cooling unit comprises a chlorous acid aqueous solution.

57. The system of any one of claims 52 to 56, wherein the poultry meat can also be used as a raw material of a chicken meat product for raw consumption.

58. A method of sterilizing poultry meat, comprising a step of contacting a chlorous acid aqueous solution with a meat raw material of a fowl for consumption.

59. The method of claim 58, comprising a step of contacting a chlorous acid aqueous solution with the fowl after removing an organ from the fowl.

60. The method of claim 58 or 59, further comprising a step of contacting a chlorous acid aqueous solution after defeathering the fowl and before removing an organ from the fowl.

61. The method of any one of claims 58 to 60, further comprising a step of contacting a chlorous acid aqueous solution after removing an organ from the fowl, contacting a chlorous acid aqueous solution with the fowl, and cleaning inside and outside of the fowl with a chlorous acid aqueous solution supplemented solution and before cooling the fowl.

62. The method of any one of claims 58 to 61, further comprising a step of contacting a chlorous acid aqueous solution after cooling the fowl and before refrigeration.

63. The method of any one of claims 58 to 62, comprising a step of cleaning inside and outside of the fowl, wherein the step of cleaning inside and outside of the fowl comprises a step of sterilizing a microorganism derived from an organ while rinsing off organ content adhering to a surface with a chlorous acid aqueous solution having a free chlorine concentration of 1 to 200 mg/L.

64. The method of any one of claims 58 to 63, wherein a microorganism adhering to a surface of a fowl is sterilized with a chlorous acid aqueous solution while cooling.

65. The method of claim 64, wherein a free chlorine concentration of a chlorous acid aqueous solution in the cooling is 1 to 200 mg/L.

66. The method of any one of claims 58 to 65, wherein the step of contacting a chlorous acid aqueous solution comprises a step of contacting a microparticulate chlorous acid aqueous solution.

67. The method of claim 66, wherein the step of contacting a chlorous acid aqueous solution comprises a step of passing the fowl through a space where a microparticulate chlorous acid aqueous solution is accumulated.

68. The method of claim 66 or 67, wherein a mean particle size of the microparticulate chlorous acid aqueous solution is 1 um to 10 um.

69. The method of any one of claims 58 to 68, wherein a free chlorine concentration of the chlorous acid aqueous solution is 1 mg/L to 200 mg/L.

70. The method of any one of claims 58 to 69, wherein the poultry meat is poultry meat that can be used not only as a conventional chicken meat raw material, but also as a raw material of a chicken meat product for raw consumption.

71. The method of any one of claims 58 to 70, wherein the fowl is a parent stock (spent hen), broiler, roaster, or jidori (free range chicken).

72. The method of any one of claims 58 to 71, wherein the method results in enterobacteriaceae adhering to the poultry meat which is negative in 25 g of a final product, Campylobacter bacteria which are negative according to a quantification method (mCCDA (SEL) medium method) and 50 MPN or less in 100 g of a final product according to a method using three sets and three tubes, Salmonella bacteria which are 0.014 cfu/g or less, and enterohemorrhagic E. coli which is 0.014 cfu/g or less.

73. The method of claim 72, wherein the enterobacteriaceae is a group of bacteria revealed based on a testing method according to the ISO methodology (21528).

74. The method of claim 72, wherein the Campylobacter bacteria are C. coli, C. concisus, C. curvus, C. fetus, C. gracilis, C. helveticus, C. hominis, C. hyointestinalis, C. insulaenigrae, C. jejuni, C. lanienae, C. lari, C. mucosalis, C. rectus, C. showae, C. sputorum, or C. upsaliensis.

75. The method of claim 72, wherein the Salmonella bacteria are S. abortusequi, S. abortusovis, S. typhisuis, S. pullorum, S. gallinarum, S. abortusbovis, S. typhi, S. paratyphi A, S. paratyphi B, S. Bongori, S. typhimurium, S. enteritidis, S. choleraesuis, S. sendai, S. oranienburg, S. chester, or S. arizonae.

76. The method of claim 72, wherein the enterohemorrhagic E. coli is E. coli O157, E. coli O26, or E. coli O111.

77. The method of any one of claims 72 to 74, wherein the Campylobacter bacteria and enterobacteriaceae comprise those derived from feces.

78. The method of any one of claims 72 to 74, wherein the Campylobacter bacteria and enterobacteriaceae comprise those derived from an organ.

79. The method of any one of claims 58 to 78, wherein a step of contacting a chlorous acid aqueous solution is performed in every step of the steps.

80. An apparatus for contacting a chlorous acid aqueous solution after defeathering a fowl and before removing an organ from the fowl.

81. An apparatus for contacting a chlorous acid aqueous solution with a fowl after removing an organ from the fowl.

82. An apparatus for contacting a chlorous acid aqueous solution after removing an organ from a fowl, contacting a chlorous acid aqueous solution with the fowl, and cleaning inside and outside of the fowl with a chlorous acid aqueous solution supplemented solution and before cooling the fowl.

83. An apparatus for contacting a chlorous acid aqueous solution after cooling the fowl and before refrigeration.

84. The apparatus of any one of claims 80 to 83, wherein the chlorous acid aqueous solution is a microparticulate chlorous acid aqueous solution.

85. The apparatus of claim 80, wherein the fowl pass through a space where a microparticulate chlorous acid aqueous solution is accumulated.

86. The apparatus of claim 84 or 85, wherein a mean particle size of the microparticulate chlorous acid aqueous solution is 1 um to 10 um.

87. The apparatus of any one of claims 80 to 86, wherein a free chlorine concentration of the chlorous acid aqueous solution is 1 mg/L to 200 mg/L.

88. A system comprising one or more of the apparatuses of any one of claims 80 to 87.

89. A system for sterilizing poultry meat, comprising an apparatus for contacting a chlorous acid aqueous solution with a meat raw material of a fowl for consumption.

90. The system of claim 89, comprising an apparatus for contacting a chlorous acid aqueous solution with the fowl after removing an organ from the fowl.

91. The system of claim 89 or 90, further comprising an apparatus for contacting a chlorous acid aqueous solution after defeathering the fowl and before removing an organ from the fowl.

92. The system of any one of claims 89 to 91, further comprising an apparatus for contacting a chlorous acid aqueous solution after removing an organ from the fowl, contacting a chlorous acid aqueous solution with the fowl, and cleaning inside and outside of the fowl with a chlorous acid aqueous solution supplemented solution and before cooling the fowl.

93. The system of any one of claims 89 to 92, further comprising an apparatus for contacting a chlorous acid aqueous solution after cooling the fowl and before refrigeration.

94. The system of any one of claims 89 to 93, wherein cleaning inside and outside of the fowl sterilizes a microorganism derived from an organ while rinsing off organ content adhering to a surface with a chlorous acid aqueous solution having a free chlorine concentration of 1 to 200 mg/L.

95. The system of any one of claims 89 to 94, wherein a microorganism adhering to a surface of a fowl is sterilized with a chlorous acid aqueous solution while cooling.

96. The system of claim 95, wherein a free chlorine concentration of a chlorous acid aqueous solution in the cooling is 1 to 200 mg/L.

97. The system of any one of claims 89 to 96 for contacting a microparticulate chlorous acid aqueous solution.

98. The system of claim 97, wherein the fowl pass through a space where a microparticulate chlorous acid aqueous solution is accumulated.

99. The system of claim 97 or 98, wherein a mean particle size of the microparticulate chlorous acid aqueous solution is 1 um to 10 um.

100. The system of any one of claims 89 to 99, wherein a free chlorine concentration of the chlorous acid aqueous solution is 1 mg/L to 200 mg/L.

101. The system of any one of claims 89 to 100, wherein the poultry meat is poultry meat that can be used not only as a conventional chicken meat raw material, but also as a raw material of a chicken meat product for raw consumption.

102. The system of any one of claims 89 to 101, wherein the fowl is a parent stock (spent hen), broiler, roaster, or jidori (free range chicken) .

103. The system of any one of claims 89 to 102, wherein the method results in enterobacteriaceae adhering to the poultry meat which is negative in 25 g of a final product, Campylobacter bacteria which are negative according to a quantification method (mCCDA (SEL) medium method) and 50 MPN or less in 100 g of a final product according to a method using three sets and three tubes, Salmonella bacteria which are 0.014 cfu/g or less, and enterohemorrhagic E. coli which is 0.014 cfu/g or less.

104. The system of claim 103, wherein the enterobacteriaceae is a group of bacteria revealed based on a testing method according to the ISO methodology (21528).

105. The system of claim 103, wherein the Campylobacter bacteria are C. coli, C. concisus, C. curvus, C. fetus, C. gracilis, C. helveticus, C. hominis, C. hyointestinalis, C. insulaenigrae, C. jejuni, C. lanienae, C. lari, C. mucosalis, C. rectus, C. showae, C. sputorum, or C. upsaliensis.

106. The system of claim 103, wherein the Salmonella bacteria are S. abortusequi, S. abortusovis, S. typhisuis, S. pullorum, S. gallinarum, S. abortusbovis, S. typhi, S. paratyphi A, S. paratyphi B, S. Bongori, S. typhimurium, S. enteritidis, S. choleraesuis, S. sendai, S. oranienburg, S. chester, or S. arizonae.

107. The system of claim 103, wherein the enterohemorrhagic E. coli is E. coli O157, E. coli O26, or E. coli O111.

108. The system of any one of claims 103 to 105, wherein the Campylobacter bacteria and enterobacteriaceae comprise those derived from feces.

109. The system of any one of claims 103 to 105, wherein the Campylobacter bacteria and enterobacteriaceae comprise those derived from an organ.

110. Use of a chlorous acid aqueous solution for the manufacture of poultry meat.

111. Use of a chlorous acid aqueous solution for the manufacture of poultry meat that can be consumed raw.

112. Use of a chlorous acid aqueous solution for sterilizing poultry meat.
